# EUROPEAN PATENT APPLICATION

(11) **EP 2 564 870 A1**
(43) Date of publication of application: **06.03.2013**
(21) Application number: 11759583.5
(22) Date of filing: 25.03.2011
(51) Int. Cl.: A61K 45/00, A61K 31/7088, A61K 38/00, A61K 39/395, A61K 48/00, A61P 31/22, A61P 43/00, C07K 14/46, C07K 16/18, C12N 15/00

(54) **PHARMACEUTICAL COMPOSITION FOR TREATMENT AND PREVENTION OF HERPESVIRUS INFECTIONS**

(30) Priority: 12.10.2010 JP 2010229537; 31.08.2010 JP 2010193101; 26.03.2010 JP 2010072717
(71) Applicant: The University of Tokyo, Bunkyo-Ku Tokyo 113-8654 (JP)
(72) Inventor: KAWAGUCHI, Yasushi, Tokyo 113-8654 (JP); ARII, Jun, Tokyo 113-8654 (JP); ARASE, Hisashi, Suita-shi Osaka 565-0871 (JP)
(74) Representative: Keirstead, Tanis Evelyne
(86) International application number: PCT/JP2011/057382
(87) International publication number: WO 2011/118779

(57) **Abstract**

An object of the present invention is to find a protein expressed in a variety of cells and functioning as a receptor for herpesvirus and provide a preventive or remedy for herpesvirus infections capable of inhibiting binding of the receptor to herpesvirus and thereby preventing entry of the virus to cells.

The present invention provides a pharmaceutical composition for preventing or treating herpesvirus infections, which composition contains a substance inhibiting the binding of glycoprotein B to a non-muscle myosin heavy chain IIA or a non-muscle myosin heavy chain.

## Description

### Technical Field

The present invention relates to a pharmaceutical composition for the treatment or prevention of herpesvirus infections.

### Background Art

Herpesviruses are DNA viruses having a linear double-stranded DNA as a genome and have a structure that a regular icosahedral nucleocapsid is enclosed in an envelope. Herpesviruses are classified into three subfamilies (alpha, beta, and gamma), but virologically important herpesviruses belong to alpha-herpesvirinae subfamily.
Alpha-herpesviruses are classified into Herpes simplex virus, Varicellovirus, Mardivirus, and Iltovirus.

Important examples of Herpes simplex virus (HSV) include Herpes simplex virus 1 (HSV-1) and Herpes simplex virus 2 having humans as a host. When HSV causes infections in humans via skin or membrane, it may be a cause of mucocutaneous diseases and could lead to fatal encephalitis.
Even if the disease is cured after first infection, viruses remain latent in the body. They are frequently reactivated and cause recurrent infections.
Important examples of Varicellovirus include porcine herpes virus 1. Porcine herpes virus 1 is also called pseudorabies virus and becomes a cause of Aujeszky's disease.

Infection of herpesvirus to host cells occurs through a complex process in which many viral and cellular factors are involved.
Herpesvirus has an envelope and two glycoproteins, that is, glycoprotein B and glycoprotein D (which may hereinafter be called "gB" and "gD", respectively) protrude from the envelope. For infection to host cells, it is necessary that these glycoproteins bind to a receptor on the cell surface and membrane fusion occurs between virus particles and cells.

It has so far been elucidated that the receptors for gD is Herpes Virus Entry Mediator (HVEM) and nectin on the cell surface (refer to, for example, Non-patent Documents 1 and 2).
The present inventors have found that Paired Immunoglobulin-like type 2 Receptor (PILR) specifically binds to gB and confirmed that HSV infects PILR expression cells; infection of PILR expression cells with HSV is inhibited by anti-PILR antibody; association between gB and PILR induces cell fusion between HSV and cells (refer to, for example, Patent Document 1). PILR is a protein expressed in immune cells such as NK cells, dendritic cells, macrophage, and mast cells.

Varicella zoster virus (VZV) belonging to Varicellovirus also has gB as an envelope protein. The present inventors have found that gB of VZV binds to Myelin Associated Glycoprotein (MAG) which is a nerve-tissue-specific molecule and confirmed that VZV infects MAG expression cells; infection of MAG expression cells with VZV is inhibited by an anti-MAG antibody; and association between gB and MAG induces membrane fusion between VZV and MAG expression cells. MAG is a cell-surface molecule specifically distributed in brain, spinal cord, and peripheral nerve tissues and is known to control the axon elongation in the nerve cells.
In addition, they have proceeded with their study and found that membrane fusion of HSV with host cells occurs via binding of gB to MAG (refer to, for example, Non-patent Document 3).

Both PILR and MAG are expressed in only very limited cultured cell lines. The expression of these gB receptors in vivo is in general limited to myeloid lineage and glial cells.
On the other hand, herpesvirus can infect various cultured cell lines in vitro, while in vivo, epithelial cells as the first infection site and nerve cells for establishment of latent infection are the most important targets of the herpesvirus.
It is therefore considered that a cell surface molecule binding to gB and functioning as a herpesvirus receptor is not limited to PILR and MAG.

One of the methods for inhibiting the binding of gB to a receptor thereof is to use an anti-gB antibody or the like to block a receptor binding site in the virus. When a substance that binds to a virus such as an anti-gB antibody is used, however, the virus itself undergoes mutation and may avoid inhibition by the substance.

As an anti-herpesvirus drug, acyclovir has been used widely. When acyclovir is phosphorylated in herpesvirus infected cells, it becomes an active form and inhibits DNA polymerase and prevents virus proliferation. It therefore acts to kill infected cells, but cannot prevent virus infection itself. It cannot therefore remove a latent infection virus from the body so that it cannot prevent recurrent infection or cannot rapidly prevent spread of infection. Moreover, herpesvirus having tolerance to aciclovir has been reported.

It has recently been reported that expression of the immediate early gene of HSV-1 is suppressed by administration of a myosin light chain kinase inhibitor or expression inhibition of myosin light chain kinase by RNAi (refer to Non-patent Document 4). According to this document, inhibition of myosin light chain kinase did not influence on the binding of virus to host cells and uptake of the virus in host cells. A hypothesis is therefore proposed that myosin II is not involved in the entry of the virus into host cells but as a result of activation of myosin II due to entry of HSV-1 into cells, an actin cell skeleton near the cell surface may be rearranged to facilitate the penetration of HSV-1 through an actin cortex.

### Prior Art Documents

### Patent Document

Patent Document 1: International Publication No. WO2008/084710

### Non-patent Documents

Non-patent Document 1: Montgomery, R.I. et al., Cell, 1996, 87:427-436
Non-patent Document 2: Geraghty, R.J. et al., Science, 1998, 280:1618-1620
Non-patent Document 3: Suenaga, T., et al., Proc. Natl. Acad. Sci. USA doi:10.1073/pnas.0913351107
Non-patent Document 4: Koithan T. et al., The 34th International Herpesvirus Workshop, July 25 to 31, 2009, Ithaca, New York, USA, Abstract 4.31

### Summary of the Invention

### Problem to be Solved by the Invention

An object of the present invention is to find a protein which is expressed in a variety of cells and functions as a herpesvirus receptor and provide a preventive or remedy of herpesvirus infections capable of inhibiting binding of the receptor to herpesvirus and thereby preventing entry of the virus into cells.

### Means for Solving the Problem

The present inventors have conducted investigations with a view to overcoming the above-described problem. As a result, it has been found that different from the hypothesis in the above-mentioned Non-patent Document 4, when herpesvirus starts infection to individuals, a non-muscle myosin heavy chain IIA and a non-muscle myosin heavy chain IIB (which may hereinafter be called "NMHC-IIA" and "NMHC-IIB", respectively) existing in all the cells including muscle cells are directed to the cell surface and C terminals of them protrude outside the cells and bind to glycoprotein gB on the surface of herpesvirus to function as an entry receptor of host cells.
It has also been confirmed that translocation of NMHC-IIA and NMHC-IIB to the vicinity of the cell surface occurs because due to herpesvirus infection, the function of myosin light chain kinase (MLCK) which controls intracellular localization of non-muscle myosin IIA and non-muscle myosin IIB (which may hereinafter be called "NM-IIA" and "NM-IIB", respectively) is enhanced and as a result, phosphorylation of a regulatory light chain (RLC) of NM-IIA and NM-IIB is increased.
It has further been found that entry of herpesvirus into cells can be prevented by inhibiting MLCK, by suppressing expression of NMHC-IIA or NMHC-IIB, by administering an anti-NMHC-IIA antibody, by administering a dominant negative mutant of MLCK, or the like, leading to the completion of the present invention.

The present invention relates to:
[1] a pharmaceutical composition for the prevention or treatment of herpesvirus infections containing, as an active ingredient, a substance which inhibits the binding of glycoprotein B to a non-muscle myosin heavy chain IIA or a non-muscle myosin heavy chain IIB;
[2] the pharmaceutical composition described above in [1], wherein the substance inhibiting the binding of glycoprotein B to a non-muscle myosin heavy chain IIA or a non-muscle myosin heavy chain IIB is a myosin ATPase activity inhibitor or a myosin light chain kinase inhibitor;
[3] the pharmaceutical composition described above in [2], wherein the myosin light chain kinase inhibitor is ML-7;
[4] the pharmaceutical composition described above in [2], wherein the myosin light chain kinase inhibitor is an MLCK pathway inhibitor;
[5] the pharmaceutical composition described above in [4], wherein the MLCK pathway inhibitor is selected from the group consisting of calmodulin antagonists, calcium chelators, and calcium antagonists:
[6] the pharmaceutical composition described above in [2], wherein the myosin light chain kinase inhibitor is a dominant negative mutant of myosin light chain kinase;
[7] the pharmaceutical composition described above in [1], wherein the substance which inhibits the binding of glycoprotein B to a non-muscle myosin heavy chain IIA or a non-muscle myosin heavy chain IIB is an antibody against the non-muscle myosin heavy chain IIA or the non-muscle myosin heavy chain IIB;
[8] the pharmaceutical composition as described above in [7], wherein the antibody binds to a peptide having an amino acid sequence as set forth in SEQ ID NO: 1 or 7;
[9] the pharmaceutical composition as described above in [7], wherein the antibody binds to a region of the non-muscle myosin heavy chain IIA or the non-muscle myosin heavy chain IIB which is exposed extracellulary upon herpesvirus infection;
[10] the pharmaceutical composition as described above in [1], wherein the substance which inhibits the binding of glycoprotein B to a non-muscle myosin heavy chain IIA or a non-muscle myosin heavy chain IIB is a substance which suppresses expression of non-muscle myosin heavy chain IIA or the non-muscle myosin heavy chain IIB;
[11] the pharmaceutical composition as described above in [10], wherein the substance which suppresses expression of the non-muscle myosin heavy chain IIA or the non-muscle myosin heavy chain IIB is selected from the group consisting of double-stranded nucleic acids having an RNAi effect, antisense nucleic acids, and ribozymes, and nucleic acids encoding them;
[12] the pharmaceutical composition as described above in [1], wherein the substance which inhibits the binding of glycoprotein B to a non-muscle myosin heavy chain IIA or a non-muscle myosin heavy chain IIB is a soluble form of the non-muscle myosin heavy chain IIA or a soluble form of the non-muscle myosin heavy chain IIB;
[13] the pharmaceutical composition as described above in any one of [1] to [12], wherein the herpesvirus is simplex herpesvirus, porcine herpesvirus 1, or cytomegalovirus;
[14] a double-stranded RNA having a base sequence as set forth in SEQ ID NO:3 and SEQ ID NO:4 and having an RNAi effect on a non-muscle myosin heavy chain IIA;
[15] a nucleic acid composed of DNA having a base sequence as set forth in SEQ ID NO:5 and encoding a double-stranded RNA having an RNAi effect against a non-muscle myosin heavy chain IIA;
[16] a double-stranded RNA having a base sequence as set forth in SEQ ID NO:9 and SEQ ID NO:10 and having an RNAi effect on a non-muscle myosin heavy chain IIB;
[17] a nucleic acid composed of DNA having a base sequence as set forth in SEQ ID NO:11 and encoding a double-stranded RNA having an RNAi effect against a non-muscle myosin heavy chain IIB;
[18] a screening method of a pharmaceutical for the prevention or treatment of herpesvirus infections, including a step of treating NMHC-IIA or NMHC-IIB expressing cells with candidate compounds;
   infecting the cells with herpesvirus; and
   measuring at least one of translocation, in the cells, of NMHC-IIA or NMHC-IIB to the vicinity of a cell membrane or entry of herpesvirus into the cells; and
[19] a screening method of a pharmaceutical for the prevention or treatment of herpesvirus infections, including bringing NMHC-IIA or NMHC-IIB, gB, and a candidate compound into contact with each other under conditions permitting binding of NMHC-IIA or NMHC-IIB to gB, and measuring the binding of NMHC-IIA or NMHC-IIB to gB.

### Effect of the Invention

The pharmaceutical composition of the present invention inhibits binding of NMHC-IIA or NMHC-IIB which functions as an entry receptor for herpesvirus in a variety of cells to glycoprotein gp on the surface of herpesvirus so that it can suppress infections of any cell in the living body with herpesvirus.
The pharmaceutical composition of the present invention prevents the entry of virus into cells so that it is not only effective for killing the virus in the infected cells but also can prevent spreading of the infection from one cell to another cell, thereby removing latent infection virus from the body and preventing recurrent infection.
A highly safe pharmaceutical with fewer side effects can be obtained by using, as a substance contained in the pharmaceutical composition of the present invention and inhibiting the binding of NMHC-IIA or NMHC-IIB to gB, a substance derived from the living body such as antibody or nucleic acid.
Moreover, when as the substance contained in the pharmaceutical composition of the present invention and inhibiting the binding of NMHC-IIA or NMHC-IIB to gB, an NM-IIA or NM-IIB inhibitor, an anti-NMHC-IIA antibody or anti-NMHC-IIB antibody, an NMHC-IIA or NMHC-IIB expression suppressant, or the like is used, the substance acts not on the herpesvirus but on the receptor (NMHC-IIA or NMHC-IIB) so that the effect would not be lost by the mutation of virus.

### Brief Description of the Drawings

[FIG. 1A] FIG. 1A shows the results of infecting MEF cells with YK711, performing immunoprecipitation with an anti-myc antibody and an anti-Flag antibody, electrophoresing the precipitate, and performing silver staining.
[FIG. 1B] FIG. 1B shows the results of infecting Vero cells with a variety of HSV-1, performing immunoprecipitation with an anti-Flag antibody or an anti-gB antibody, and subjecting the precipitate to immunoblotting with an anti-NMHC-IIA antibody.
[FIG. 2A] FIG. 2A shows the results of analyzing the binding of the soluble C-terminal fragment of NMHC-IIA to a variety of HSV-1 by using flow cytometry.
[FIG. 2B] FIG. 2B shows the results of analyzing the binding of a gB transfectant or gD transfectant of HSV-1 to the soluble C-terminal fragment of NMHC-IIA by using flow cytometry.
[FIG. 3A] FIG. 3A shows the results of infecting Vero cells with HSV-1 and observing the intracellular localization of NMHC-IIA with a fluorescence microscope.
[FIG. 3B] FIG. 3B shows the results of biotinylating the surface protein of cells infected with HSV-1, performing immunoprecipitation with avidin beads, and then carrying out immunoblotting with an anti-NMHC-IIA antibody.
[FIG. 3C] FIG. 3C shows the results of infecting cells with each of gB expressing HSV-1 and gH expressing HSV-1, biotinylating the cell surface protein, and then precipitating with an anti-Flag antibody.
[FIG. 4A] FIG. 4A shows the results of studying the HSV-1 infection of cells, which have been pretreated with an MLCK inhibitor ML-7, by flow cytometry.
[FIG. 4B] FIG. 4B shows the results of observing, with a fluorescence microscope, the concentration of NMHC-IIA in the vicinity of the cell membrane of cells pretreated with an MLCK inhibitor ML-7 at the time of HSV-1 entry.
[FIG. 4C] FIG. 4C shows the results of making a similar test to that of FIG. 4A by using an influenza virus.
[FIG. 5A] FIG. 5A shows the results of analyzing, by flow cytometry, HSV-1 infection to Vero cells pretreated with an anti-NMHC-IIA serum.
[FIG. 5B] FIG. 5B shows the results of analyzing, by flow cytometry, HSV-1 infection to CHO-hNectin-1 cells pretreated with an anti-NMHC-IIA serum.
[FIG. 5C] FIG. 5C shows the results of analyzing, by flow cytometry, HSV-1 infection to CHO-PILRa cells pretreated with an anti-NMHC-IIA serum.
[FIG. 6A] FIG. 6A shows the results of analyzing, by flow cytometry, HSV-1 infection to shRNA-mediated NMHC-IIA knockdown cells.
[FIG. 6B] FIG. 6B shows the results of analyzing, by flow cytometry, influenza virus infection to shRNA-mediated NMHC-IIA knockdown cells.
[FIG. 6C] FIG. 6C shows the results of membrane fusion assay between NMHC-IIA knockdown cells and HSV-1.
[FIG. 6D] FIG. 6D shows the results of VSV envelope G protein-mediated membrane fusion assay in NMHC-IIA knockdown cells.
[FIG. 7A] FIG. 7A shows the results of confirming overexpression of NMHC-IIA in HL60/NMHC-IIA cells by using western blotting method.
[FIG. 7B] FIG. 7B shows the results of analyzing HSV-1 infection to NMHC-IIA overexpressing cells by using flow cytometry.
[FIG. 7C] FIG. 7C shows the results of observing HSV-1 infection to NMHC-IIA overexpressing cells by using a fluorescence microscope.
[FIG. 7D] FIG. 7D shows the results of analyzing, by flow cytometry, the influence of an anti-NMHC-IIA serum on the HSV-1 infection to NMHC-IIA overexpressing cells.
[FIG. 7E] FIG. 7E shows the results of analyzing, by flow cytometry, pseudorabies virus infection to NMHC-IIA overexpressing cells and inhibition of the infection by an anti-NMHC-IIA serum.
[FIG. 8] FIG. 8 is a schematic view showing the preparation process of YK711 and YK712.
[FIG. 9A] FIG. 9A shows the results of analyzing, by flow cytometry, HSV-1 entry into cells pretreated with acyclovir.
[FIG. 9B] FIG. 9B shows the results of analyzing, by flow cytometry, HSV-1 entry into cells pretreated with ML-7.
[FIG. 9C] FIG. 9C shows the results of analyzing, by flow cytometry, HSV-1 entry into cells pretreated with an anti-NMHC-IIA serum.
[FIG. 10] FIG. 10 shows the results of infecting Vero cells with HSV-1 in the presence or absence of an MLCK inhibitor (ML-7) and measuring phosphorylation of RLC by immunoblotting.
[FIG. 11A] FIG. 11A shows the results of infecting Vero cells with HSV-1, leaving the resulting cells in the presence or absence of an MLCK inhibitor (BAPTA-AM) and then analyzing intracellular localization of NMHC-IIA by immunofluorescent method using an anti-NMHC-IIA antibody.
[FIG. 11B] FIG. 11B shows the results of infecting Vero cells with HSV-1 in the presence or absence of an MLCK inhibitor (BAPTA-AM) and measuring phosphorylation of RLC by immunoblotting.
[FIG. 11C] FIG. 11C shows the results of infecting Vero cells with HSV-1 GFP in the presence or absence of BAPTA-AM at the indicated concentrations and then measuring average fluorescence intensity by flow cytometry.
[FIG. 11D] FIG. 11D shows the results of infecting Vero cells with an influenza virus in the presence or absence of BAPTA-AM at the indicated concentrations and then measuring average fluorescence intensity by flow cytometry.
[FIG. 12A] FIG. 12A shows the results of infecting, Vero cells, which have been transformed with a mock expression plasmid (Vector) or an expression plasmid of dominant negative mutant of MCLK (Dn-MLCK), with HSV-1 and measuring phosphorylation of RLC by immunoblotting using an anti-phosphorylate RLC antibody or an anti-RLC antibody.
[FIG. 12B] FIG. 12B shows the results of determining, from the results of FIG. 12A, an amount of phosphorylated RLC protein relative to total RLC protein mass.
[FIG. 12C] FIG. 12C shows the results of infecting Vero cells, which have been transformed with Vector or Dn-MLCK, with wild-type HSV-1 GFP and measuring average fluorescence intensity by flow cytometry.
[FIG. 12D] FIG. 12D shows the results of infecting Vero cells, which have been transformed with Vector or Dn-MLCK, with a wild-type influenza virus and measuring average fluorescence intensity by flow cytometry.
[FIG. 13] FIG. 13 shows the results of administering, in advance, an MLCK inhibitor ML-7 to a murine corneal infection model, inoculating it with wild-type HSV-1 and studying virus titer in tear, keratitis symptoms, and survival rate.
[FIG. 14] FIG. 14 shows the results of administering, to a murine corneal infection model, an MLCK inhibitor ML-7 and wild-type HSV-1 simultaneously and studying a survival rate.
[FIG. 15] FIG. 15 shows the results of administering, in advance, an MLCK inhibitor BAPTA-AM to a murine corneal infection model, inoculating the model with wild-type HSV-1, and studying a survival rate.
[FIG. 16A] FIG. 16A shows the results of studying the expression of NMHC-IIA and NMHC-IIB proteins in Vero cells and Cos-1 cells.
[FIG. 16B] FIG. 16B shows the results of infecting Cos-1 cells with YK711 or YK712, performing immunoprecipitation with an anti-Flag antibody, and performing immunoblotting of the precipitate with an anti-NMHC-IIB antibody.
[FIG. 16C] FIG. 16C shows the results of infecting Cos-1 cells with wild-type HSV-1(F), performing immunoprecipitation with an anti-Flag antibody or an anti-gB antibody, and performing immunoblotting of the precipitate with an anti-NMHC-IIB antibody.
[FIG. 17A] FIG. 17A shows the results of analyzing, by flow cytometry, binding of the gB transfectant or gD transfectant of HSV-1 to the soluble C-terminal fragment of NMHC-IIB.
[FIG. 17B] FIG. 17B shows the results of analyzing, by flow cytometry, binding of the gB transfectant or gD transfectant of HSV-1 to control Fc (CD200).
[FIG. 18A] FIG. 18A shows the results of performing or not performing ML-pretreatment with ML-7, biotinylating the surface of Cos-1 cells infected with HSV-1, performing immunoprecipitation with avidin beads, and then performing immunoblotting with an anti-NMHC-IIB antibody.
[FIG. 18B] FIG. 18B shows the results of studying, by flow cytometry, the HSV-1 infection to Cos-1 cells pretreated with an MLCK inhibitor ML-7.
[FIG. 18C] FIG. 18C shows the results of performing a similar test to that of FIG. 18B by using an influenza virus.
[FIG. 19A] FIG. 19A shows the results of immunoblotting performed to confirm shRNA-mediated knockdown of NMHC-IIB in Cos-1 cells.
[FIG. 19B] FIG. 19B shows the results of analyzing, by flow cytometry, ²HSV-1 infection to shRNA-mediated NMHC-IIB-knockdown cells.
[FIG. 19C] FIG. 19C shows the results of analyzing, by flow cytometry, influenza virus infection to shRNA-mediated NMHC-IIB-knockdown cells.
[FIG. 19D] FIG. 19D shows the results of membrane fusion assay between NMHC-IIB-knockdown cells and HSV-1.
[FIG. 19E] FIG. 19E shows the results of VSV envelope G protein-mediated membrane fusion assay in NMHC-IIB-knockdown cells.
[FIG. 20A] FIG. 20A shows the results of confirming NMHC-IIB overexpression in IC21/NMHC-IIB cells by western blotting method.
[FIG. 20B] FIG. 20B shows the results of observing HSV-1 infection to NMHC-IIB overexpressing cells by using a fluorescence microscope.
[FIG. 20C] FIG. 20C shows the results of analyzing HSV-1 infection to NMHC-IIB overexpressing cells by using flow cytometry.
[FIG. 21A] FIG. 21A shows the results of infecting Cos-1 cells with HSV-1 GFP in the presence or absence of BAPTA-AM at the indicated concentrations and then measuring average fluorescence intensity by flow cytometry.
[FIG. 21B] FIG. 21B shows the results of infecting Cos-1 cells with an influenza virus in the presence or absence of BAPTA-AM at the indicated concentrations and then measuring average fluorescence intensity by flow cytometry.
[FIG. 22] FIG. 22 shows the results of studying HSV-2 infection to cells pretreated with ML-7 by flow cytometry.
[FIG. 23] FIG. 23 shows the results of analyzing HSV-1 infection to cells pretreated with an anti-NMHC-IIArod mouse serum or an anti-NMHC-IIBrod mouse serum by flow cytometry.
[FIG. 24] FIG. 24 shows the results of analyzing HSV-2 infection to cells pretreated with an anti-NMHC-IIArod mouse serum or an anti-NMHC-IIBrod mouse serum by flow cytometry.
[FIG. 25] FIG. 25 shows the results of observing HCMV infection to cells pretreated with an anti-NMHC-IIArod mouse serum or an anti-NMHC-IIBrod mouse serum by a fluorescence microscope.
[FIG. 26] FIG. 26 shows the results of measuring HCMV infection to cells pretreated with an anti-NMHC-IIArod mouse serum or an anti-NMHC-IIBrod mouse serum by FACS.
[FIG. 27] FIG. 27 shows the results of measuring HSV-2 infection of NMHC-IIA knockdown cells by flow cytometry.
[FIG. 28] FIG. 28 shows the results of measuring HSV-2 infection of NMHC-IIB knockdown cells by flow cytometry.
[FIG. 29] FIG. 29 shows the results of measuring HSV-2 infection of NMHC-IIA overexpressing cells by flow cytometry.
[FIG. 30] FIG. 30 shows the results of measuring HSV-2 infection of NMHC-IIB overexpressing cells by flow cytometry.

### Mode for Carrying out the Invention

The mode for carrying out the present invention will next be described.
The pharmaceutical composition of the present invention is used for the treatment or prevention of herpesvirus infections and characterized in containing a substance which inhibits binding of glycoprotein B to a non-muscle myosin heavy chain IIA or a non-muscle myosin heavy chain IIB.

Herpesvirus is, as described above, an animal DNA virus and Herpesviridae viruses are classified into three subfamilies, that is, the alphaherpesvirinae, betaherpesvirinae and gammaherpesvirinae. As described above, alphaherpesvirinae is classified further into Herpes simplex virus, Varicellovirus, Mardivirus, and Iltovirus and typical ones include HSV-1, HSV-2, varicella/zoster virus, porcine herpes virus 1 (pseudorabies virus), and bovine herpesvirus.
Viruses belonging to the betaherpesvirinae include human cytomegalovirus (HCMV), while as viruses belonging to the gammaherpesvirinae, EB virus, Kaposi's sarcoma-associated herpesvirus, and the like are known.
As viruses belonging to the gammaherpesvirinae, Epstein-Barr virus (EB virus) of Lymphocryptovirus genus is typical.
The pharmaceutical composition according to the present invention may be used for any one of alpha, beta, and gamma herpesvirus infections. For example, it is used for infections with alphaherpesviruses such as HSV-1, HSV-2, or porcine herpesvirus 1 (pseudorabies virus), infections with betaherpesviruses such as HCMV, and infections with gammaherpesviruses such as EB virus.
Herpes simplex virus is one of herpesviruses and as described above, is classified into HSV-1 and HSV-2. HSV-1 causes mainly labial herpes, may cause herpes stomatitis, herpes keratitis, herpes simplex encephalitis, and the like, and remains latent in trigeminal ganglion. HSV-2 may mainly cause genital herpes, neonatal herpes, herpes meningitis, herpes myelitis, or the like and remains latent in spinal ganglion.
Cytomegalovirus remains inapparent in most of infections at birth or during infancy and also remains inapparent in many infections in children or adults, but it happens to cause hepatitis or mononucleosis. Infection with it becomes serious in immunodeficient patients such as AIDS patients and malignancy patients. Ganciclovir is used for the treatment of pneumonia or retinitis caused by this virus.
Porcine herpes virus 1 is also called Aujeszky's disease virus and infection with it is a notifiable infectious disease. It infects many animals including pigs and in pigs, infection is mainly inapparent. Animals other than pigs however show neurological symptoms and after an acute course, they result in death.
With regard to EB virus, many people become a carrier thereof due to inapparent infection in childhood. When adults are infected with it, it may be a cause of infectious mononucleosis, which is presumed to be a cause of Burkitt's lymphoma or upper pharynx cancer.

The term "infection" as used herein means either one of percutaneous or transmucous entry of a virus into a living body or association of a glycoprotein on the virus surface with a receptor (entry receptor) on the cell surface, followed by entry into the cells by membrane fusion. The term "herpesvirus infection" as used herein means the condition where the virus has entered into the living body irrespective of symptoms and it embraces persistent infection, inapparent infection, and latent infection.

The term "treatment or prevention of herpesvirus infections" is used in its broadest meaning and more specifically, it means amelioration of one or more symptoms related to herpesvirus infectious diseases or inhibition of worsening of the symptom(s); suppression of generation of postinfectious symptoms; inhibition (retardation or termination) of virus infection to cells in the living body; reduction in the number of viruses in the living body; and the like.

Glycoprotein B (gB) is one of glycoproteins present on the surface of herpesvirus. Examples of glycoproteins similar to it include gD, gH, and gL, of which gB and gD bind to a receptor on the cell surface. In order to cause membrane fusion and virus infection to cells, both gB and gD should bind to a receptor on the cell surface.

The term "non-muscle myosin heavy chain IIA (NMHC-IIA)" means a non-muscle myosin IIA (NM-IIA) heavy chain subunit which is one of non-muscle myosin II (NM-II) heavy chain isoforms.
The term "non-muscle myosin heavy chain IIB (NMHC-IIB)" means a non-muscle myosin IIB (NM-IIB) heavy chain subunit which is one of NM-II heavy chain isoforms.
NM-II is a protein existing in every cell including a muscle cell and plays an important role in cell movement such as cytokinesis, cell migration, and alteration of cellular morphology. NM-II is, similar to muscle myosin, composed of six subunits, that is, two heavy chains, two essential light chains, and two regulatory light chains and has two spherical head portions and elongated tail portions (rod domains). The head portion has a motor domain containing an ATPase active site and an actin binding site and a neck which is a light-chain binding site. The rod domain has an α-helical coiled-coil structure and is involved in association of NM-II to form a bipolar filament. The C terminal (rod domain end) does not have a coiled-coil structure.
When a regulatory light chain Ser19 is phosphorylated by Ca²⁺/calmodulin-dependent myosin light chain kinase (MLCK), NM-II of vertebrates has an increased ATPase activity and can form a filament.
Vertebrates have three NM-II heavy chain isoforms and they form homodimers to become NM-IIA, NM-IIB, NM-IIC, respectively. Of these, NM-IIA and NM-IIB are much similar in their amino acid sequence and properties and it is reported that NM-IIA and NM-IIB heavy chain knockout mice result in embryonic lethality. In addition, NMHC-IIA and NMHC-IIB have about 80% homology.
As shown later in Examples, judging also from that there exist, in the living body, NMHC-IIA predominant cells (for example, epithelial cells), NMHC-IIB predominant cells (for example, nerve cells) and cells in which only either one of NMHC-IIA or NMHC-IIB has been expressed, they are presumed to have a similar function (refer to, for example, Bresnik AR, Curr Opin Cell Biol. 1999 Feb;11(1):26-33.; Sellers JR, Biochim Biophys Acta. 2000 Mar 17;1496(1):3-22, and the like).

As described above, there was a report on the relationship between NM-II and HSV-1 infection, but according to it, NM-II was not involved in the entry to host cells of HSV-1 but NM-II was activated after entry of HSV-1 to the cells (Non-patent Document 4).
The present inventors however have demonstrated that as shown later in Examples, when herpesvirus starts infection to individuals, a Ca²⁺/calmodulin complex activates MLCK to direct NMHC-IIA and NMHC-IIB to the cell surface; and have confirmed that when NMHC-IIA or NMHC-IIB binds to gB on the virus surface, membrane fusion between virus and cell occurs, leading to entry of the virus to the cell.
The present inventors have thought that since this binding is inhibited by an antibody prepared using, as an antigen, a fragment containing the C-terminal of NM-IIA, NMHC-IIA or NMHC-IIB translocates to the cell surface upon herpesvirus infection and the C terminal thereof exposed outside the cell functions as an entry receptor for herpesvirus.
The term "entry receptor" as used herein means a receptor which causes membrane fusion between herpesvirus and cells by binding to the herpesvirus.

In this description, no particular limitation is imposed on a substance inhibiting the binding of gB to NMHC-IIA or NMHC-IIB (which may hereinafter be called simply "binding inhibitor") insofar as it directly or indirectly inhibits the binding of gB to NMHC-IIA or NMHC-IIB and any substance such as low molecular compounds, nucleic acids, peptides, and proteins can be used. Specific examples include NM-IIA or NM-IIB ATPase activity inhibitors, inhibitors of myosin light chain kinase (MLCK), anti-NMHC-IIA antibodies, anti-NMHC-IIB antibodies, and NMHC-IIA or NMHC-IIB expression inhibitors. These substances will next be described.

(NMHC-IIA or NMHC-IIB ATPase activity inhibitors or MLCK inhibitors)
The NMHC-IIA or NMHC-IIB ATPase activity inhibitor or MLCK inhibitor in the present invention changes intracellular localization of NMHC-IIA or NMHC-IIB and prevents translocation of NMHC-IIA or NMHC-IIB to the cell surface when cells are infected with herpesvirus.
Examples of the ATPase activity inhibitor include Blebbistatin.

As the MLCK inhibitor, inhibitors selectively inhibiting myosin light chain kinase can be used and in addition, serine/threonine kinase inhibitors, cell-permeable protein kinase inhibitors, and the like can be used insofar as they inhibit myosin light chain kinase activity.
Specific examples include, but not limited to, A3, Calphostin C, Goe6976, Goe7874, HA1077, Hypericin, K-252a, KT5823, ML-7, ML-9, Piceatannol, Staurosporine, W-5, W-7, W-12, W-13, and Wortmannin. Of these, ML7 having high selectivity to MLCK is preferred.
Moreover, since MLCK is Ca²⁺/calmodulin dependent, inhibitors inhibiting an MLCK pathway can also be used as the MLCK inhibitor. The term "MLCK pathway" as used herein means a series of reactions including formation of a complex between Ca²⁺ and calmodulin, activation of MLCK by binding with the complex, and phosphorylation of RLC by MLCK. Examples of the MLCK pathway inhibitor include inhibitors of the formation of a Ca²⁺/calmodulin complex and inhibitors of the binding of a Ca²⁺/calmodulin complex to MLCK (such as calmodulin antagonists, calcium chelators, and calcium antagonists).
Examples of the calcium chelators include, but not limited to, BAPTA (O,O'-bis(2-aminophenyl)ethylene glycol-N,N,N',N' -tetraacetic acid tetraacetoxy ester) and BAPTA-AM obtained by acetoxymethyl esterification of all the carboxyl groups of BAPTA to facilitate uptake of it in cells.
As a function inhibitor of MLCK, MLCK dominant negative mutants may be used. The mutants are preferably expressed in the living body by administering a vector containing a gene encoding the dominant negative mutants. Such a vector can be prepared in a conventional manner by those skilled in the art. Examples include a method of inserting a DNA encoding the dominant negative mutant of MLCK into a cloning site of a plasmid or the like; and a method of introducing both a plasmid having a backbone sequence of an adenovirus vector and a shuttle plasmid having a homological sequence thereto at both ends of a DNA encoding the MLCK dominant negative mutant into cells or Escherichia coli to prepare a virus vector by homologous recombination.

### (Anti-NMHC-IIA antibody, anti-NMHC-IIB antibody)

The anti-NMHC-IIA antibody of the present invention specifically binds to NMHC-IIA and as a result, it inhibits the binding of NMHC-IIA to gB. The anti-NMHC-IIB antibody of the present invention specifically binds to NMHC-IIB and as a result, it inhibits the biding of NMHC-IIB to gB.
The present inventors have confirmed that an antibody prepared using, as an antigen, a fragment containing the C-terminal of NMHC-IIA (peptide corresponding to positions from 1665 to 1960 of NMHC-IIA; SEQ ID NO:1) inhibits the binding of NMHC-IIA to herpesvirus gB. This indicates that when NMHC-IIA translocates to the vicinity of the cell surface by virus infection, the C terminal of it protrudes outside the cell membrane and binds to gB. Similarly, NMHC-IIB which translocates to the vicinity of the cell surface by virus infection and protrudes the C terminal outside the cell membrane can also be inhibited from binding to gB by an antibody prepared using, as an antigen, a fragment containing the C terminal.

The anti-NMHC-IIA antibody of the present invention is not particularly limited insofar as it inhibits the binding of NMHC-IIA to gB. For example, an antibody binding to an extracellularly exposed region of NMHC-IIA upon herpesvirus infection is preferred. Such an antibody can bind to a binding region of NMHC-IIA to gB and thereby inhibit the binding of NMHC-IIA to gB.
Specific examples include antibodies that bind to a region of from about 1 to about 500 amino acids, preferably from about 100 to about 400 amino acids, more preferably from about 200 to 300 amino acids in the vicinity of the C terminal of NMHC-IIA (for example, 1000 amino acids from the C terminal). An antibody binding to a region corresponding to positions from 1665 to 1960 of NMHC-IIA can be given as one example.

Although no particular limitation is imposed on the anti-NMHC-IIB antibody of the present invention insofar as it inhibits the binding of NMHC-IIB to gB, it is preferably an antibody binding to an extracellularly exposed region of NMHC-IIB upon herpesvirus infection. Such an antibody can inhibit binding of NMHC-IIB to gB by binding to a binding region of NMHC-IIB to gB.
Specific examples include antibodies that bind to a region of from about 1 to about 500 amino acids, preferably from about 100 to about 400 amino acids, more preferably from about 200 to 300 amino acids in the vicinity of the C terminal of NMHC-IIB (for example, 1000 amino acids from the C terminal). An antibody binding to a region corresponding to positions from 1672 to 1976 of NMHC-IIB can be given as one example.

The term "antibody" as used herein embraces also an antibody fragment. The anti-NMHC-IIA antibody or anti-NNHC-IIB antibody in the present invention may be a monoclonal antibody, a polyclonal antibody, a recombinant antibody, a human antibody, a humanized antibody, a chimeric antibody, a single chain antibody, a Fab fragment, a F(ab')₂ antibody, scFv, a double specific anybody, a synthetic antibody, or the like.
These antibodies can be prepared in a known manner by those skilled in the art. For example, a monoclonal antibody can be obtained by isolating antibody producing cells from non-human mammals immunized with NMHC-IIA or NMHC-IIB, fusing the antibody producing cells with myeloma cells or the like to form hybridoma, and purifying an antibody produced by this hybridoma. A polyclonal antibody can be obtained from a serum of animals immunized with NMHC-IIA or NMHC-IIB.
The NMHC-IIA or NMHC-IIB used for immunization may be either human-derived or another animal-derived one. Either an entire length or fragment may be used, which can be determined as needed by those skilled in the art. When a fragment is used, a fragment of a region of NMHC-IIA or NMHC-IIB which is exposed extracellulary upon herpesvirus infection can be used. For example, the fragment may be consisting of from about 1 to about 500 amino acids, preferably from about 100 to about 400 amino acids, more preferably from about 200 to 300 amino acids in the vicinity of the C terminal of NMHC-IIA or NMHC-IIB (for example, 1000 amino acids from the C terminal). For example, a fragment consisting of an amino acid sequence as set forth in SEQ ID NO:1 having 296 amino acids at positions from 1665 to 1960 (which may hereinafter be called "C terminal fragment of NMHC-IIA") or a fragment of an amino acid sequence as set forth in SEQ ID NO: 7 having 305 amino acids at positions from 1672 to 1976 (which may hereinafter be called "C terminal fragment of NMHC-IIB") may be used.

If a non-human monoclonal antibody capable of efficiently inhibiting the binding of NMHC-IIA or NMHC-IIB to herpesvirus can be obtained, it can be reproduced by gene recombination. For example, after total RNA is prepared from hybridoma, which produces the anti-NMHC-IIA monoclonal antibody, by a standard procudure and mRNA encoding the anti-NMHC-IIA antibody is prepared using a commercially available kit, cDNA is synthesized using a reverse transcriptase to obtain a DNA encoding the anti-NMHC-IIA antibody. By transfecing an expression vector containing such a DNA to appropriate host cells and culturing the resulting cells under appropriate conditions, the anti-NMHC-IIA antibody can be expressed. The anti-NMHC-IIB antibody can also be expressed similarly.

Alternatively, DNAs encoding CDR regions can be obtained by PCR using the above-mentioned cDNA as a template. By making use of such DNAs encoding CDR regions, a human antibody or humanized antibody can be prepared in a conventional manner by gene recombination. For example, a DNA encoding a human antibody can be obtained by synthesizing a DNA designed to connect DNAs encoding CDR regions derived from a non-human antibody with a DNA encoding the frame work region of a human antibody by using PCR and connecting with a DNA encoding a human antibody constant region further.
In a known manner (such as a method using a restriction enzyme), such a DNA is inserted into an expression vector (for example, plasmid, retrovirus, adenovirus, adeno-associated virus (AAV), plant virus such as cauliflower mosaic virus and tobacco mosaic virus, cosmid, YAC, or EBV-derived episome) and the expression vector is transfected into appropriate host cells to obtain a transformant. The expression vector may further contain a promoter for regulating the expression of an antibody gene, a replication origin, a selective marker gene, and the like. The promoter and the replication origin can be selected as needed, depending on the kind of host cells and vector.
Next, the transformant thus obtained is cultured under appropriate conditions to express an anti-NMHC-IIA antibody or anti-NMHC-IIB antibody which is a human antibody.
Examples of the host cells usable include eukaryotic cells such as mammalian cells (CHO cells, COS cells, myeloma cells, HeLa cells, Vero cells, and the like), insect cells, plant cells, and fungal cells (Saccharomyces, Aspergillus, and the like) and prokaryotic cells such as Escherichia coli and Bacillus subtilis.

The antibody thus expressed can be isolated/purified by using, in combination, known methods (for example, affinity column using protein A or the like, other chromatography column, filter, ultrafiltration, salting-out, dialysis, etc.).
When the anti-NMHC-IIA antibody or anti-NMHC-IIB antibody of the present invention is a low molecular antibody such as Fab fragment, F(ab')₂ fragment, or scFv, expression can be achieved according to the above method by using a DNA encoding a low molecular antibody or it may be obtained by treating an antibody with an enzyme such as papain or pepsin.

### (NMHC-IIA or NMHC-IIB expression inhibitor)

The expression inhibitor of NMHC-IIA or NMHC-IIB in the present invention is not particularly limited insofar as it is a substance capable of suppressing intracellular expression of NMHC-IIA or NMHC-IIB. Examples include double-stranded nucleic acids having an RNAi effect, antisense nucleic acids, and ribozymes, and nucleic acids encoding them. By inhibiting the expression itself of NMHC-IIA or NMHC-IIB, it is possible to reduce NMHC-IIA or NMHC-IIB that functions as a receptor upon herpesvirus infection and thereby prevent herpesvirus from invading the cells.

The RNAi effect is a sequence-specific gene expression suppressing mechanism induced by a double-stranded nucleic acid. It has high target specificity and is highly safe because it makes use of a gene expression suppressing mechanism originally present in the living body.
Examples of the double-stranded nucleic acid having an RNAi effect include siRNA. When used for mammalian cells, siRNA is usually a double-stranded RNA composed of from about 19 to 30 bases, preferably from about 21 to 25 bases. As the NMHC-IIA or NMHC-IIB expression inhibitor in the present invention, a longer double-stranded RNA which will be siRNA as a result of cleavage by an enzyme (Dicer) may be used. Usually in the double-stranded nucleic acid having an RNAi effect, one of the strands has a base sequence complementary to a portion of a target nucleic acid and the other strand has a sequence complementary thereto. The double-stranded nucleic acid having an RNAi effect usually has two protruding bases (overhangs) at the 3' terminal of each strand, but it may be a blunt end type having no overhang. For example, a blunt end RNA with 25 bases is also suited for use in vivo, because it has an advantage that it minimizes the activation of an interferon response gene, prevents an off target effect derived from a sense chain, and has very high stability in the serum.
The double-stranded nucleic acid having an RNAi effect can be designed in a known manner based on the base sequence of the target gene. The double-stranded nucleic acid having an RNAi effect may be a double-stranded RNA or a DNA-RNA chimeric double-stranded nucleic acid, or it may be an artificial nucleic acid or a nucleic acid subjected to various modifications.

The double-stranded nucleic acid having an RNAi effect in the present invention preferably targets a region of a gene encoding NMHC-IIA including a base sequence as set forth in SEQ ID NO:2. Examples of such a double-stranded nucleic acid include a double-stranded RNA composed of an RNA having a base sequence as set forth in SEQ ID NO:3 and an RNA having a base sequence as set forth in SEQ ID NO:4.
The double-stranded nucleic acid having an RNAi effect in the present invention preferably targets a region of a gene encoding NMHC-IIB including a base sequence as set forth in SEQ ID NO:8. Examples of such a double-stranded nucleic acid include a double-stranded RNA composed of an RNA having a base sequence as set forth in SEQ ID NO:9 and an RNA having a base sequence as set forth in SEQ ID NO:10.

The antisense nucleic acid is a single-stranded nucleic acid having a base sequence complementary to a target gene (basically, mRNA which is a transcription product) and usually having a length of from 10 bases to 100 bases, preferably a length of from 15 bases to 30 bases. Gene expression is inhibited by introducing the antisense nucleic acid into cells to hybridize to the target gene. It is not necessary that the antisense nucleic acid is completely complementary to the target gene insofar as it produces an expression inhibitory effect of the target gene. The antisense nucleic acid can be designed as needed by those skilled in the art by using known software or the like. The antisense nucleic acid may be any one of DNA, RNA, and DNA-RNA chimelle or it may be modified.

The ribozyme is a nucleic acid molecule that catalytically hydrolyzes a target RNA and is composed of an antisense region having a sequence complementary to the target RNA and a catalytic center region involved in cleavage reaction. The ribozyme can be designed as needed by those skilled in the art in a known manner. The ribozyme is typically an RNA molecule, but a DNA-RNA chimeric molecule may be used.

Nucleic acids encoding any one of the double-stranded nucleic acid having an RNAi effect, the antisense nucleic acid, and the ribozyme can be used as an expression inhibitor of the NMHC-IIA or NMHC-IIB of the present invention. When a vector containing such a nucleic acid is introduced into cells, the double-stranded nucleic acid having an RNAi effect, the antisense nucleic acid, and the ribozyme are expressed to exhibit the NMHC-IIA or NMHC-IIB expression suppressing effects.
As the nucleic acid encoding the double-stranded nucleic acid having an RNAi effect, DNAs respectively encoding the double strands or a DNA encoding a single-stranded nucleic acid obtained by linking a double-stranded nucleic acid via a loop may be used. In the latter case, the single-stranded RNA produced by intracellular transcription has a hairpin-shaped structure with its complementary portion being hybridized in the molecule. This RNA is called shRNA (short hairpin RNA). When shRNA migrates to the cytoplasm, it becomes a double-stranded RNA as a result of cleavage of its loop portion by an enzyme (Dicer) and exhibits an RNAi effect.
The above-mentioned double-stranded RNA having the base sequence as set forth in SEQ ID NO:2 as a target can also be obtained by using a DNA as set forth in SEQ ID NO:5 and expressing shRNA in the cells. The double-stranded RNA having the base sequence as set forth in SEQ ID NO:8 as a target can also be obtained by using a DNA having a base sequence as set forth in SEQ ID NO:11 and expressing shRNA in the cells.

### (Soluble form of NMHC-IIA or soluble form of NMHC-IIB)

The soluble-form of NMHC-IIA of the present invention binds to herpesvirus gB outside cells and as a result, inhibits the binding of NMHC-IIA on the cell surface to gB.
The soluble form of NMHC-IIA is a molecule having binding ability to gB and containing the whole or a part of NMHC-IIA or mutant thereof.
When the soluble form of NMHC-IIA contains a part of NMHC-IIA, it may contain any part insofar as it has binding ability to gB, for example, a rod domain or C-terminal fragment of NMHC-IIA.
The soluble form of NMHC-IIA may be a fusion protein between the whole or part of NMHC-IIA and another soluble form of a protein. As such a soluble form of a protein, for example, IgG protein or Fc region thereof is preferably employed.
The soluble form of NMHC-IIA can be formed by those skilled in the art by using gene recombination.

Also, the soluble form of NMHC-IIB of the present invention binds to herpesvirus gB outside cells and as a result, inhibits the binding of the NMHC-IIB on the cell surface to gB.
The soluble form of NMHC-IIB is a molecule having binding ability to gB and containing the whole or a part of NMHC-IIB or mutant thereof.
When the soluble form of NMHC-IIB contains a part of NMHC-IIB, it may contain any part insofar as it has binding ability to gB, for example, a rod domain or a C-terminal fragment of NMHC-IIB.
The soluble form of NMHC-IIB may be a fusion protein between the whole or part of NMHC-IIB and another soluble form of a protein. As such a soluble form of a protein, for example, IgG protein or Fc region thereof is preferably employed.
The soluble form of NMHC-IIB can be formed by those skilled in the art by using gene recombination.

### (Pharmaceutical composition)

The pharmaceutical composition of the present invention can be administered orally or parenterally, and systematically or locally. For example, intravenous injection such as infusion, intramuscular injection, intraperitoneal injection, subcutaneous injection, suppository, enema, or oral enteric coated drug can be selected. An administration method can be selected as needed, depending on the age and symptoms of a patient.

The pharmaceutical composition of the present invention may contain a pharmaceutically acceptable carrier such as preservative or stabilizer. The pharmaceutically acceptable carrier is a material which can be administered with the substance (active ingredient) inhibiting the binding of gB to NMHC-IIA or NMHC-IIB. The pharmaceutically acceptable carrier is not particularly limited insofar as it is pharmacologically and pharmaceutically acceptable. Examples include, but not limited to, water, saline, phosphate buffer, dextrose, glycerol, pharmaceutically acceptable organic solvents such ethanol, collagen, polyvinyl alcohol, polyvinylpyrrolidone, carboxyvinyl polymer carboxymethyl cellulose sodium, sodium polyacrylate, sodium alginate, water-soluble dextran, carboxymethyl starch sodium, pectin, methyl cellulose, ethyl cellulose, xanthan gum, gum Arabic, casein, agar, polyethylene glycol, diglycerin, glycerin, propylene glycol, petrolatum, paraffin, stearyl alcohol, stearic acid, human serum albumin, mannitol, sorbitol, lactose, surfactants, excipients, flavoring agents, preservatives, stabilizers, buffers, suspending agents, tonicity agents, binders, disintegrants, lubricants, fluidity accelerators, taste corrigents, and the like.

The pharmaceutical composition can be formulated into typical medical preparations. These medical preparations are obtained as needed using the above-mentioned carrier. No particular limitation is imposed on the form of the medical preparation and it is selected as needed, depending on the purpose of treatment. Typical examples include tablets, pills, powders, liquids, suspensions, emulsions, granules, capsules, suppositories, and injections (liquids, suspensions, or emulsions). These preparations may be produced in a conventional manner.

When the pharmaceutical composition of the present invention contains a nucleic acid, preparations can be obtained by enclosing the nucleic acid in a carrier such as liposome, high-molecular micelle, or cationic carrier. A nucleic acid carrier such as protamine may be used. An affected part is preferably targeted by an antibody or the like bound to such a carrier. In addition, the retention in the blood can be improved by binding cholesterol or the like to the nucleic acid. When the pharmaceutical composition of the present invention contains a nucleic acid encoding siRNA or the like which is expressed in the cells after administration, the nucleic acid inserted into a virus vector such as retrovirus, adenovirus, or Sendai virus or a non-virus vector such as liposome may be administered in the cells.

The amount of the active ingredient contained in the pharmaceutical composition of the present invention can be determined as needed by those skilled in the art, depending on the kind of the active ingredient. For example, the administration amount of an anti-NMHC-IIA antibody or an anti-NMHC-IIB antibody is from 0.025 to 50 mg/kg, preferably from 0.1 to 50 mg/kg, more preferably from 0.1 to 25 mg/kg, still more preferably from 0.1 to 10 mg/kg or 0.1 to 3 mg/kg, but the administration amount is not limited thereto.
The pharmaceutical composition of the present invention can be administered to humans or mammals other than humans (such as mice, rats, rabbits, dogs, pigs, cows, horses, and monkeys) in order to prevent or treat herpesvirus infections.

### (Therapeutic method)

The present invention embraces a method of preventing or treating herpesvirus infections, the method being characterized by administering a therapeutically effective amount of the pharmaceutical composition of the present invention. The term "therapeutically effective amount" as used herein means an amount that ameliorates one or a plurality of symptoms related to the herpesvirus infection or prevent worsening of the symptoms, suppresses occurrence of postinfectious symptoms, prevents (retards or terminates) infection of cells with viruses in the living body, or decrease the number of viruses in the living body.
The therapeutic method of the present invention is used for therapeutic objects such as humans and mammals other than humans (for example, mice, rats, rabbits, dogs, pigs, cows, horses, and monkeys).

### (Screening method)

The present invention also provides a method of screening for pharmaceuticals for the prevention or treatment of herpesvirus infections.
In one embodiment, the screening method of the present invention includes:
treating NMHC-IIA or NMHC-IIB expressing cells with candidate compounds;
infecting the cells with herpesvirus; and
measuring at least one of translocation of NMHC-IIA or NMHC-IIB to the vicinity of the cell membrane or entry of herpesvirus into the cells.
The NMHC-IIA or NMHC-IIB expressing cells may be cells which originally express it or cells obtained by transforming cells, which do not originally express it or scarcely express it, with an expression vector containing a gene encoding NMHC-IIA or NMHC-IIB. As the cells which originally express NMHC-IIA, for example, Vero cells can be used, while as the cells which originally express NMHC-IIB, Vero cells and Cos-1 cells can be used. As the cells from which almost no expression of NMHC-IIA is observed, HL60 cells can be used, while as the cells from which almost no expression of NMHC-IIB is observed, IC21 cells can be used.

The candidate compounds may be any substance such as low molecular compounds, high molecular compounds, peptides, proteins, and nucleic acids. The treatment of the candidate compounds may be performed prior to infection, in parallel with infection, or after infection. The treatment method can be determined by those skilled in the art, depending on the properties of the candidate compounds.

The translocation of NMHC-IIA or NMHC-IIB to the vicinity of the cell membrane can be observed, as shown later in Examples, by detecting NMHC-IIA or NMHC-IIB bound to a fluorescence-labeled anti-NMHC-IIA antibody or an anti-NMHC-IIB antibody by using a fluorescence microscope. The entry of herpesvirus into the cells can be observed easily by a fluorescence microscope, as shown later in Examples, by infecting with a recombinant herpesvirus having an expression cassette of a green fluorescence protein (GFP) or an enhanced green fluorescent protein (EGFP).
The pharmaceutical thus selected inhibits NMHC-IIA or NMHC-IIB from functioning as a gB receptor for herpesvirus and is useful as a preventive or remedy of herpesvirus infections.

In another embodiment, the screening method of the present invention includes:
bringing NMHC-IIA or NMHC-IIB, gB, and candidate compounds into contact with each other under conditions permitting binding of NMHC-IIA or NMHC-IIB to gB, and
measuring the binding of NMHC-IIA or NMHC-IIB to gB.
As NMHC-IIA or NMHC-IIB, that isolated may be used or NMHC-IIA or NMHC-IIB expressing cells may be used. A partial peptide or soluble form containing a binding site to gB may be used. As gB, a virus having, on the surface thereof, gB may be used or alternatively, as shown later in Example 1, cells infected with a virus may be used.
The step of measuring the binding of NMHC-IIA or NMHC-IIB to gB may be performed after selected, as needed by those skilled in the art, from known methods for detecting the interaction between proteins such as immunoprecipitation and flow cytometry.

### Examples

The present invention will hereinafter be described in detail based on Examples, but it should be noted that the present invention is not limited by them.

### [Virus]

The following are various viruses used in the examples.

### YK711 and YK712

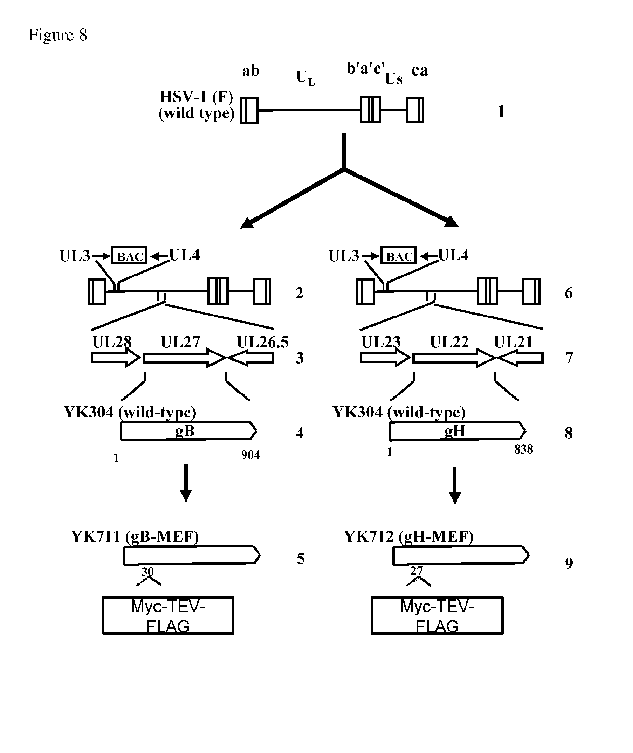

YK711 and YK712 are recombinant HSV-1s that respectively express Myc-TEV-Flag(MEF)tag-labeled gB (MEF-gB) and Myc-TEV-Flag(MEF)tag-labeled gH (MEF-gH). According to the method of Kato, et al. (Kato, A. et al. J Virol 82, 6172-6189 (2008)), they were prepared using E. coli GS1783 (Jarosinski, K. et al. J Virol 81, 10575-87.) containing pYEbac102 through two-step Red-mediated mutagenesis (Jarosinski, K. et al. J Virol 81, 10575-87.).
A schematic view of the preparation process is shown in FIG. 8.

### Wild type HSV-1(F), gB-deficient virus, and revertant virus thereof (in which the gB sequence deleted in the gB-deficient virus has been restored)

They were prepared according to the methods of Satoh and et al. and Kawaguchi and et al. (Kawaguchi, Y. et al. J Virol 73, 4456-60., Satoh, T.et al. Cell 132, 935-44.).

### HSV-1 GFP

HSV-1 GFP is a recombinant HSV-1 having, in the intergenic region between UL3 gene and UL4 gene, an enhanced green fluorescent protein (EGFP) expression cassette under control of Egr-1 promoter.

### HSV-2 GFP

HSV-2 GFP is a recombinant HSV-2 containing an enhanced green fluorescent protein (EGFP) expression cassette under control of a cytomegalovirus promoter in the intergenic region between UL50 gene and UL51 gene and a bacmid (J. Virol. 83: 11624-11634, 2009).

### PRV GFP

Recombinant pseudorabies virus PRV151 having EGFP under control of human cytomegalovirus in gG locus. Provided by Dr. L. W. Enquist.

HSV-1 GFP and PRV GFP, similar to a wild-type virus, grow in cultured cells and only infected cells express a fluorescent protein.

### HCMV ADsubU21.5

GFP expression HCMV (Wang D. et al., PNAS 101: 16642-16647, 2004) was provided by Dr. T. Shenk.

### [Fusion protein, peptide, etc]

Fusion proteins and peptides used in Examples were expressed using the following plasmids.

### pGEX-NMHC-IIArod

pGEX-NMHC-IIArod is a plasmid for producing a fusion protein (GST-NMHC-IIArod) of glutathione S-transferase and the C-terminal fragment of NMHC-IIA (SEQ ID NO:1). A coding region of the C terminal fragment of NMHC-IIA was constructed by amplifying from pEGFP-ARF296 (Sato, M.et al. Mol Biol Cell 18, 1009-17.) by PCR and clogning the resulting DNA fragment into pGEX-4T3 (GE Healthcare) in flame with GST.

### pGEX-NMHC-IIBrod

pGEX-NMHC-IIBrod is a plasmid for producing a fusion protein (GST-NMHC-IIBrod) of glutathione S-transferase and the C-terminal fragment of NMHC-IIB. A coding region of the C terminal fragment of NMHC-IIB was constructed by amplifying from pEGFP-BRF305 (Sato, M.et al. Mol Biol Cell 18, 1009-17.) by PCR and cloning the resulting DNA fragment into pGEX-4T3 (GE Healthcare) in flame with GST.

### pME-Ig-NMHC-IIArod and pME-Ig-NMHC-IIBrod

pME-Ig-NMHC-IIArod and pME-Ig-NMHC-IIBrod are plasmids for producing fusion proteins (NMHC-IIArod-Ig and NMHC-IIBrod-Ig) of Ig and the C-terminal fragment of NMHC-IIA or NMHC-IIB, which is soluble form of NMHC-IIA or soluble form of NMHC-IIB, respectively.
pME-Ig-NMHC-IIArod was prepared in a similar manner to that of pGEX-NMHC-IIArod except that a modified pME18S expression vector was used instead of pGEX-4T-3. The modified pME18S expression vector contains a mouse CD150 leader segment at the N terminal and a Fc segment of human IgG1 at the C terminal. In this Fc segment, in order to reduce binding affinity to cellular Fc receptors, leucines at positions 266 and 267 were mutated into alanine and glutamin, respectively, and in order to reduce the binding affinity to HSV-1 Fc receptors (gE), histidine at position 467 was mutated into arginine.
pME-Ig-NMHC-IIBrod was prepared in a similar manner. For pME-Ig-NMHC-IIBrod, however, a coding region of the C-terminal fragment of NMHC-IIB (SEQ ID NO:7) was amplified from pEGFP-BRF305 (Sato, M.et al. Mol Biol Cell 18, 1009-17.) by PCR.

### pMxs-NMHC-IIA-puro

An open leading frame of NMHC-IIA from plasmid 11347 (product of Addgene) was cloned into pMxs-puro (Morita, S. et al. Gene Ther 7, 1063-6.). This plasmid is called "pMxs-NMHC-IIA-puro".

### Flag-MYH10 Expression vector

This vector was prepared according to the method of Uchiyama Y et al. Proc Natl Acad Sci USA. 2010 May 18;107(20):9240-5.

### pEP98-gB, pPEP99-gD, pPEP101-gL, and pPEP100-gH (HSV-1 glycoprotein expression plasmids)

pEP98-gB, pPEP99-gD, pPEP101-gL, and pPEP100-gH are plasmids for expressing gB, gD, gL, and gH of HSV-1. They were obtained from Northwestern University (Pertel, P. E.et al. Virology 279, 313-24).

### pT7EMCLuc

pT7EMCLuc was used for the determination of a fusion efficiency. It is a plasmid having pCAGT7 encoding T7 RNA polymerase and a firefly luciferase gene under the control of a T7 promoter (Okuma, K.et al. Virology 254, 235-44.).

### pSSSP-NMHC-IIA, pSSSP-NMHC-IIB and pSSSP-Cre

For the production of a stable cell line expressing shRNA against NMHC-IIA or NMHC-IIB, pSSSP-NMHC-IIA and pSSS-NMHC-IIB were constructed according to the following method.
DNA (SEQ ID NO:5) encoding shRNA against NMHC-IIA was cloned into the BbsI site and the EcoRI site of pmU6. The BamHI-EcoRI fragment (containing a U6 promoter and a sequence encoding shRNA against NMHC-IIA) of the resulting plasmid was cloned into the BamHI and EcoRI sites of pSSSP to obtain pSSSP-NMHC-IIA. The pSSSP is a derivative of a retrovirus vector pMX containing a puromycin resistance gene.
The pSSSP-NMHC-IIB was prepared in a similar manner to pSSSP-NMHC-IIA except that a DNA having a base sequence as set forth in SEQ ID NO:11 was used as a DNA encoding shRNA against NMHC-IIB.
As a control, pSSSP-Cre encoding shRNA against Cre recombinase was prepared according to Haraguchi, T., et al. FEBS Lett 581, 4949-54.

### [Cells and media]

Following cells were used in Examples.
CHO-hPILRα cells and CHO-hNectin-1 cells: they are transformants that stably express human PILRα and human nectin-1 respectively (Arii, J. et al. J Virol. 83, 4520-7.).
HL60/NMHC-IIA cells and HL60/puro cells: H60 cells having puromycin resistance and obtained by transduction with recombinant retroviruses containing MXs-NMHC-IIA and pMxs-puro, respectively. More specifically, plat-GP cells were co-transfected with pMxs-NMHC-IIA-puro or pMxs-puro in combination with pMDG. Two days after transfection, the supernatant was collected. The HL60 cells were transduced by infection with retrovirus-containing supernatants of the transfected plat-GP cells. To a maintenance medium was added 0.5 µg/ml of puromycin and cells thus transduced were selected.
IC21/NMHC-IIB cells and IC21/puro cells: IC21 cells having puromycin resistance and obtained by trasnduction with recombinant retroviruses containing a Flag-MYH10 expression vector and pMxs-puro, respectively. More specifically, IC21 cells were transduced with a Flag-MYH10 expression vector or pMxs-puro. From two days after the transduction, the resulting transductant was cultured on a maintenance medium containing 0.25 µg/ml of puromycin and the transduced cells were selected.
A 199 medium, a Ham F-12 medium, a PMI1640 medium supplemented with 1%FCS, a 199 medium supplemented with 1%FCS were used for virus infection of various cells such as Vero cells, CHO cells, HL60 cells, IC21 cells, and HEL cells.

### [Antibody]

The following are antibodies used in Examples.
Mouse monoclonal antibodies against gB (1105), Flag (M2) and Myc (PL14): purchased from Goodwin Institute, Sigma, and MBL, respectively.
Rabbit polyclonal antibody against C-terminal of NMHC-IIA: purchased from Sigma. This antibody recognizes 11 amino acids (SEQ ID NO:6) at the C terminal of NMHC-IIA as an epitope.
Rabbit polyclonal antibody (anti-NMHC-IIA serum) against the C-terminal fragment of NMHC-IIA used in Example 4: A rabbit was immunized with GST-NMHC-IIArod obtained by expressing the above-mentioned pGEX-NMHC-IIArod in E.coli, followed by purification according to a conventional protocol (MBL). The serum of the immunized rabbit was used as an anti-NMHC-IIArod polyclonal antibody. A control rabbit serum was purchased from MBL.
Rabbit polyclonal antibody against the C terminal of NMHC-IIB: purchased from Sigma. This antibody recognizes 12 amino acids (SEQ ID NO:12) at the C terminal (positions from 1965 to 1976) of NMHC-IIB as an epitope.
Mouse polyclonal antibody against the C terminal of NMHC-IIA and NMHC-IIB: Mice were immunized with GST-NMHC-IIArod and GST-NMHC-IIBrod, respectively and immunized mouse serums were used, respectively, as an anti-NMHC-IIArod mouse serum and NMHC-IIBrod mouse serum.
An anti-phosphorylated RLC antibody and an anti-RLC antibody were purchased from Cell signaling Technology.

### Example 1: Search for novel HSV entry receptor that binds to gB

### <Search for entry receptor in mouse embryonic fibroblasts (MEF cells)>

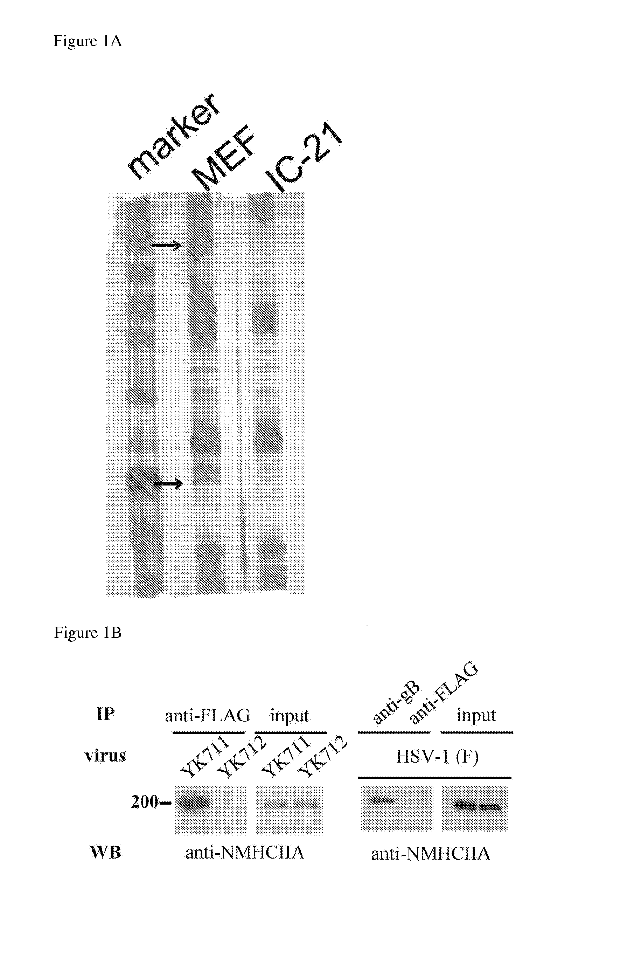

MEF cells or IC21 cells (mouse macrophage-like cells) were infected with YK711 at 4°C for 2 hours, and the resulting cells were transferred to 37°C for 2 minutes and harvested. After treatment with a phosphate buffered saline (PBS) containing 2 mM DTSSP (Piers) at 4°C for 2 hours, they were lysed in a RIPA buffer (1% NP-40, 0.1% Sodium Deoxycholate, 0.1% SDS, 150 mM NaCl, 10 mM Tris-HCl [pH7.4], 1 mM EDTA).
The supernatant obtained after centrifugation was subjected to first immunoprecipitation using an anti-myc monoclonal antibody (MBL) and the immunoprecipitate was reacted with AcTEV protease (Invitrogen). In addition, the above-mentioned supernatant was subjected to second immunoprecipitation using an anti-Flag monoclonal antibody (Sigma). The immunoprecipitate was separated by electrophoresis in a denaturing gel and visualized by silver staining.
This makes it possible to detect a protein that binds to gB in fibroblasts which are widely distributed in the living body.
The results are shown in FIG. 1A.
Next, the bands (arrows) only found in MEF cells were excised and digested in the gel with trpsin, then analyzed by a mass spectrometer. As a result of mass analysis, one of the bands was identified as MMHC-IIA.

### <Expression of NMHC-IIA and NMHC-IIB>

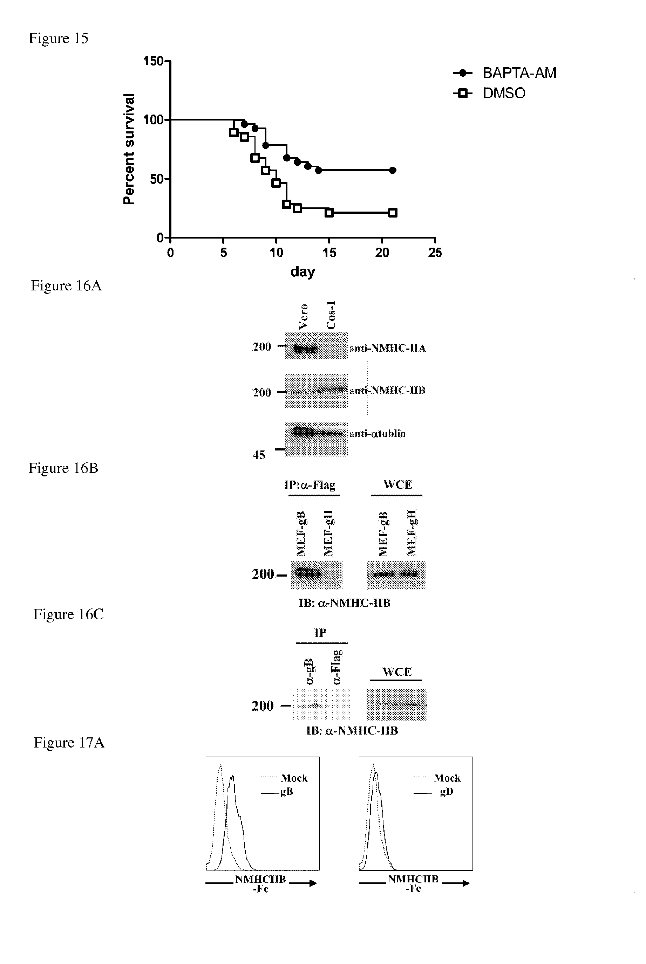

In Vero cells and Cos-1 cells, expression of NMHC-IIA and NMHC-IIB having an analogous function to that of NMHC-IIA were detected, respectively, by using an anti-NMHC-IIA antibody and an anti-NMHC-IIB antibody. The results are shown in FIG. 16A. In Vero cells, both were expressed, while in Cos-1 cells, only NMHC-IIB was expressed.

### <Immunoprecipitation>

In order to find an HSV entry receptor other than PILR and MAG, a method using a tandem affinity purification using a cross linker that does not have membrane permeability and a proteomics technology using mass spectrometry (Oyama, M. et al. Mol Cell Proteomics 8, 226-31.) in combination was employed. The following is a specific method.
Vero cells were infected with YK711, YK712 or HSV-1(F) at 4°C for 2 hours. The cells were transferred to 37°C for 2 minutes and harvested. The cells were then washed with PBS, and lysed in a TNE buffer (1% NP-40, 150mM NaCl, 10mM Tris-HCl [pH7.8], 1mM EDTA) containing a proteinase inhibitor cocktail. The supernatant obtained after centrifugation was precleared by incubation with protein A-sepharose beads at 4°C for 30 minutes. After short-time centrifugation, the supernatant thus obtained was reacted with an anti-Flag antibody or an anti-gB antibody at 4°C for 2 hours. Then, protein A-sepharose beads were added. The resulting mixture was reacted at 4°C for 1 hour while rotating. The immunoprecipitate was collected by short-time centrifugation, washed extensively with a TNE buffer, and analyzed by immunoblotting using an anti-NMHC-IIA antibody.
The results are shown in FIG. 1B.
In a cell lysate of Vero cells infected with YK711 expressing MEF-gB or wild-type HSV-1(F), NMHC-IIA was coprecipitated with MEF-gB or wild-type gB.
FIGS. 16B and 16C show the results of a similar experiment except that Vero cells were replaced by Cos-1 cells and analyzing the resulting immunoprecipitate by immunoblotting with an anti-NMHC-IIB antibody. FIG. 16B shows the results of infection with YK711 or YK712, while FIG. 16C shows the results of infection with wild-type HSV-1(F).
It has been confirmed that as in the case of NMHC-IIA, in the lysate of Cos-1 cells infected with the YK711 that expresses MEF-gB or with wild-type HSV-1(F), NMHC-IIB is coprecipitated with MEF-gB or wild-type gB and NMHC-IIB also binds to gB.

### <Binding of wild-type HSV-1(F), gB-deficient virus, or a revertant virus to Soluble C-terminal fragment of NMHC-IIA>

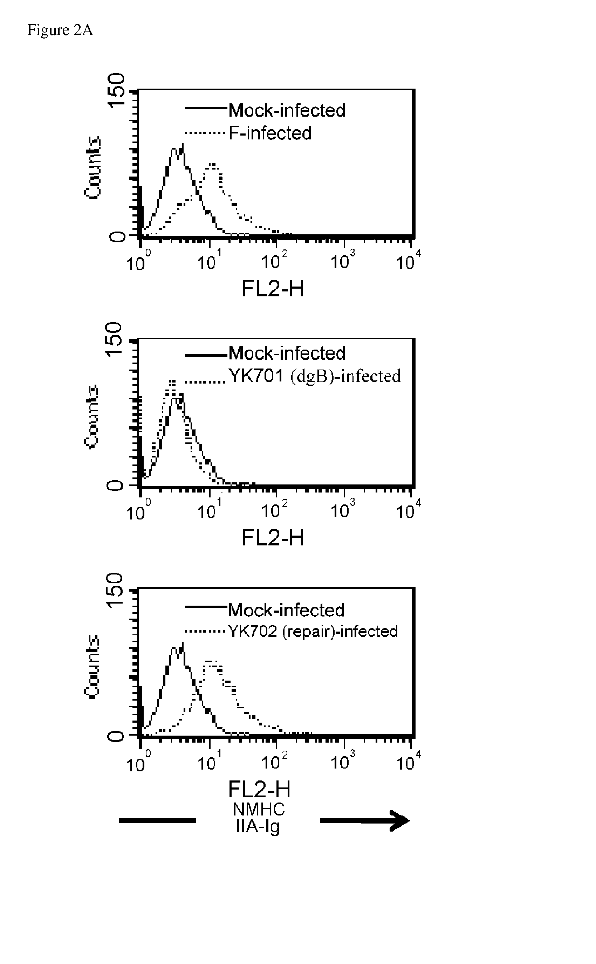

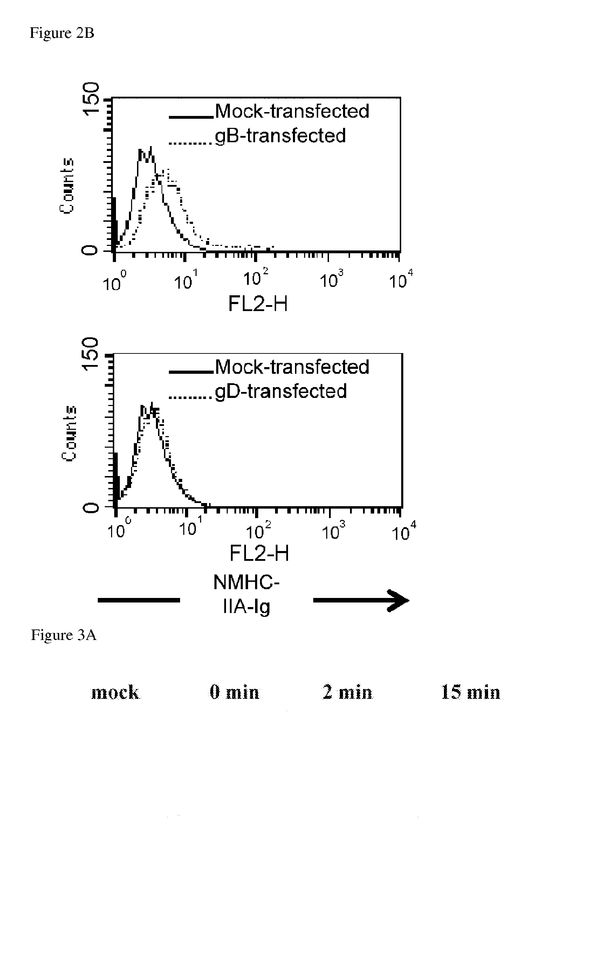

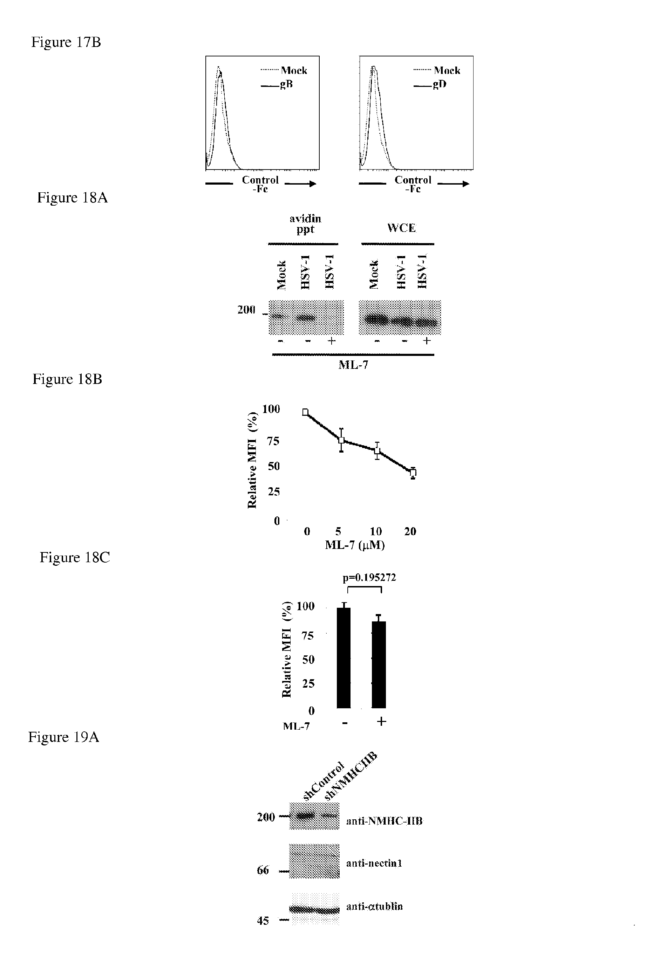

NMHC-IIArod-Ig was produced in Cos-1 cells. 293T cells were infected with HSV-1. Twelve hours later, the infected cells were collected (A). To other 293T cells was introduced a HSV-1 glycoprotein expression plasmid by using lypofectamine. Eighteen hours later, the resulting cells were collected (B). The cells were reacted with NMHC-IIArod-Ig for 30 minutes on ice. After washing with PBS containing 2%FCS, the cells were reacted with a secondary antibody (PE-labeled anti-human IgG antibody) on ice for 30 minutes. The cells were washed with PBS containing 2% FCS again and analyzed using a flow cytometer.
Results of (A) and (B) are shown in FIGS. 2A and 2B, respectively.
The gB-deficient virus infected cells (YK701(dgB)-infected) were not recognized by NMHC-IIArod-Ig (fusion protein of the C terminal fragment of NMHC-IIA and Ig) (middle graph of IG. 2A). On the other hand, the wild-type HSV-1(F) infected cells (F-infected) and the cells infected with the revertant virus of the gB-deficient virus expressing wild-type gB (YK702(repair)-infected) were recognized by NMHC-IIArod-Ig (upper and lower panels of FIG. 2A).
Consistent with these results, the gB transfectant of HSV-1 was recognized by NMHC-IIArod-Ig and the gD transfectant of HSV-1 was not recognized (FIG. 2B).
These results have suggested that the C-terminal region of NMHC-IIA interacts with gB of HSV-1.
Next, NMHCIIBrod-Ig was produced in Cos-1 cells and an HSV-1 glycoprotein expression plasmid was introduced into 293T cells by using lipofectamine, and a similar test to that described above (B) was conducted on NMHC-IIB. The results are shown in FIGS. 17A and 17B. Cells expressing gB on their surface was stained with NMHC-IIBrod-Ig, suggesting that the C-terminal region of NMHC-IIB also interacts with gB of HSV-1.

### Example 2: Intracellular translocation of NMHC-IIA upon HSV-1 entry

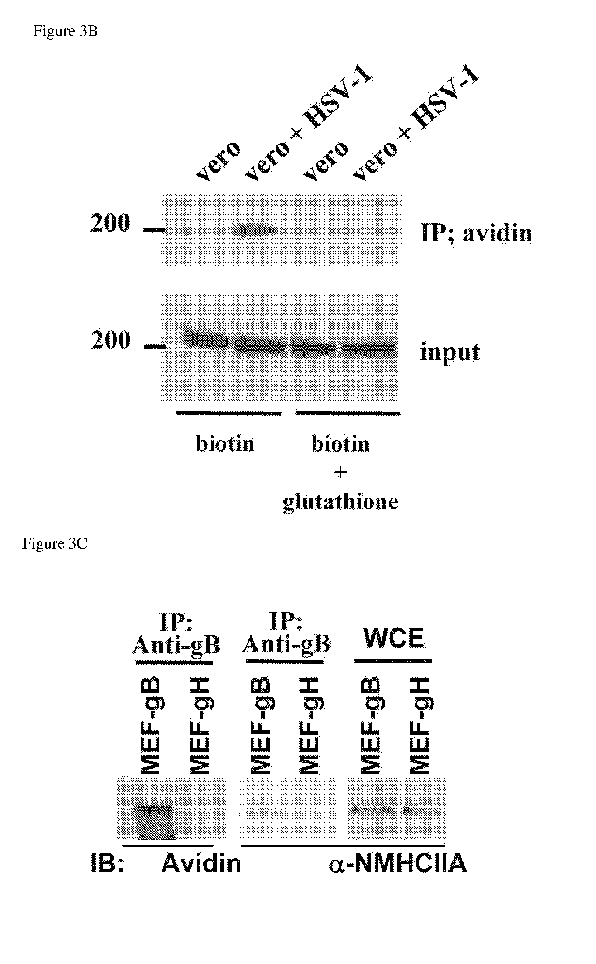

Vero cells were infected with wild-type HSV-1(F) at 4°C for 2 hours. Zero minute, two minutes, and fifteen minutes after transfer of the resulting cells to 37°C, the intracellular localization of NMHC-IIA was analyzed by immunofluorescence method (a FITC-labeled secondary antibody was used) using an anti-NMHC-IIA antibody.
In the mock-infected cells and the cells infected with HSV-1(F) at 4°C (after 0 minute), NMHC-IIA was distributed throughout the cytoplasm. When HSV-1 started entry (2 minutes and 15 minutes after transfer to 37°C), the concentration of NMHC-IIA in the vicinity of the cell membrane showed a significant increase (FIG. 3A).
The surface protein of the mock-infected cells or the cells which were left for 15 minutes after infection with wild-type HSV-1(F) at 4°C for 2 hours and transfer to 37°C was biotinylated. Immunoprecipitation was performed with avidin beads, followed by immunoblotting with an anti-NMHC-IIA antibody.
More specifically, Vero cells were infected with HSV-1(F) at MOI=5 at 4°C for 2 hours. The cells were transferred to 37°C, washed four times with ice-cold BPS after 2 minutes and 15 minutes, and biotylated twice each for 15 minutes by using a cleavable sulfo-NHS-SS-Biotin (Pierce).
After washing the biotylated cells once with ice-cold DMEM containing 0.2% BSA and then washing twice with PBS containing 10% FCS, the resulting cells were subjected to mock treatment or were treated with a freshly prepared reducing solution (15.5 mg of glutathione/ml, 75mM NaCl, 0.3% NaOH, and 10% calf serum) twice at 4°C for 20 minutes in order to remove the remaining biotin labeling from the protein at the cell surface.
After washing twice with DMEM containing 0.2% BSA and quenching the free SH-group in 5 mg/ml iodoacetamide (in PBS) containing 1% BSA, the cells were collected and solubilized in a RIPA buffer containing a proteinase inhibitor cocktail. Avidin beads were precipitated and immunoblotting was conducted using an anti-NMHC-IIA antibody.
The results are shown in FIG. 3B.
Expression of NMHC-IIA on the surface of normal Vero cells and an increase in the amount of NMHC-IIA on the cell surface due to virus entry were confirmed.
Moreover, in the cells infected with MEF-gB-expressing HSV-1 (YK711), the NMHC-IIA coprecipitated with the anti-Flag antibody was biotinylated, but in the cells infected with MEF-gH-expressing HSV-1 (YK712), a protein having a molecular weight equal to that of the biotinylated NMHC-IIA was not detected (FIG. 3C).
These results show that due to the HSV-1 entry, the concentration of NMHC-IIA at the cell surface increases and NMHC-IIA at the cell surface interacts with gB.
The property of NMHC-IIA that the expression at the cell surface increases within 15 minutes after HSV-1 entry cannot be observed for entry receptors of HSV-1 so far reported and is therefore unique to NMHC-IIA. This property is consistent with the previous report that there is a time lag of from 10 minutes to 15 minutes from the adsorption of HSV-1 to cells to the entry and explains the phenomenon. Such a time lag cannot be observed in a vaccinal virus or an influenza virus.

### Example 3-1: Inhibition of HSV-1 infection by control of intracellular localization of NMHC-IIA

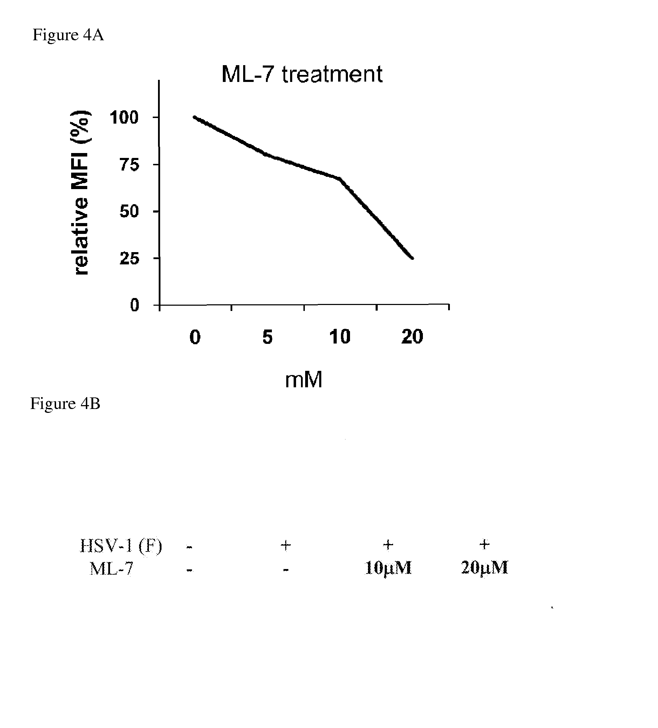

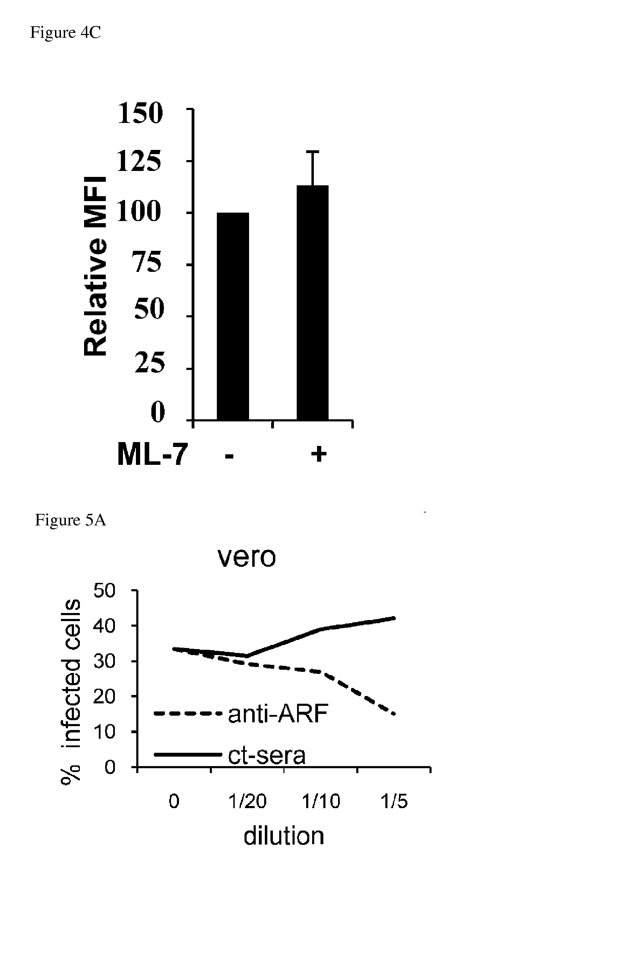

Intracellular localization of NM-IIA is partially controlled through phosphorelation of a regulatory light chain (RLC), a subunit of NM-IIA, by myosin light chain kinase (MLCK).
The influence of ML-7, a specific inhibitor of MLCK, on the rearrangement of NMHC-IIA was investigated. More specifically, Vero cells pretreated with various concentrations of ML-7 for 30 minutes were inoculated with HSV-1 GFP at MOI of 1 by using a 24-well plate in the presence of the same concentrations of ML-7. After removal of the inoculum, the cells were refed with the medium containing the same concentrations of ML-7.
Five hours, six hours, or twelve hours after infection, the cells were analyzed using a fluorescence microscope (Olympus IX71) or analyzed by FacsCalibur while using a Cell Quest software (Becton Dickinson).
In addition, a similar test was performed using an influenza virus.
The results are shown in FIG. 4.
An increase in the concentration of NMHC-IIA in the vicinity of the cell membrane upon virus entry was inhibited by ML-7 (FIG. 4B).
Infection with HSV-1 GFP was also inhibited dose-dependently by ML-7 (FIG. 4A), while influenza virus infection was not influenced by ML-7 (FIG. 4C).
These results have shown that control of NM-IIA including translocation of NMHC-IIA to the cell surface upon HSV-1 entry is required for efficient HSV-1 infection.
Translocation of NMHC-IIA is presumed to be controlled by the adjustment of a signal pathway that occurs immediately after virus entry. The HSV-1 entry induces a drastic increase in the calcium concentration in the cell membrane and in the cells (Cheshenko, N. et al. Mol Biol Cell 18, 3119-30), which causes MLCK-mediated phosphorylation of NM-II RLC.
The fact demonstrated herein that a specific inhibitor of MLCK that phosphorylates NM-II RLC and controls localization of NM-II inhibits translocation of NMHC-IIA upon virus entry and HSV-1 infection supports the hypothesis that activation of a calcium signaling pathway caused by HSV-1 entry induces translocation of NMHC-IIA and mediates virus entry achieved by interaction with gB.

In a similar manner to Example 2, the Cos-1 cells pretreated or not pretreated with ML-7 were infected with wild-type HSV-1(F). After biotinylation of the cell surface proteins and immunoprecipitation with avidin beads, immunoblotting was conducted using an anti-NMHC-IIB antibody.
The results are shown in FIG. 18A. The concentration of NMHC-IIB in the vicinity of the cell membrane increased by HSV-1 infection, but when pretreated with ML-7, the concentration decreased significantly.
In a similar manner to Example 3, Cos-1 cells pretreated for 30 minutes with various concentrations of ML-7 were inoculated with HSV-1 GFP at MOI=1 by using a 24-well plate in the presence of the same concentrations of ML-7. After removal of the inoculum, the cells were refed with the medium containing the same concentrations of ML-7. Further, a similar test was made using an influenza virus instead of HSV-1 GFP.
The results are shown in FIGS. 18B and 18C. As shown in FIG. 18B, HSV-1 infection was dose-independently inhibited by ML-7. The influenza virus infection was not influenced by ML-7 (FIG. 18C). Such a phenomenon was observed also in Cos-1 cells which had expressed only NMHC-IIB, showing that NMHC-IIB also translocates to the cell surface and functions as a receptor for HSV-1 entry upon HSV-1 entry.

### Example 3-2: Inhibition of HSV-2 infection by control of intracellular localization of NMHC-IIA

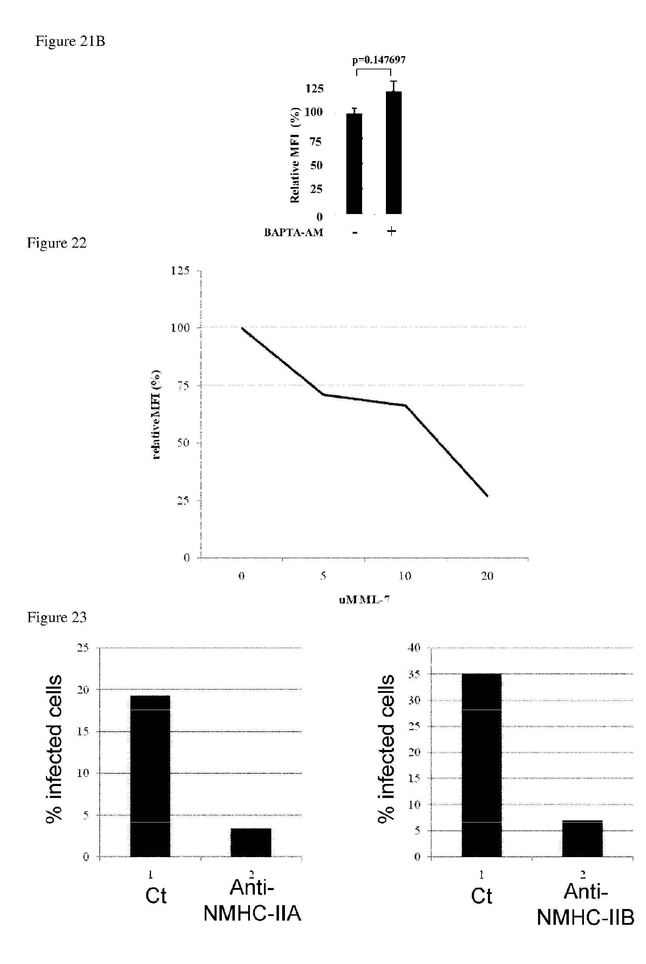

A similar test to that of Example 3-1 was made on HSV-2 GFP by using Vero cells.
The results are shown in FIG. 22. HSV-2 GFP infection was dose-independently inhibited by ML-7.

### Example 4-1: Inhibition of HSV-1 infection by anti-NMHC-IIA antibody

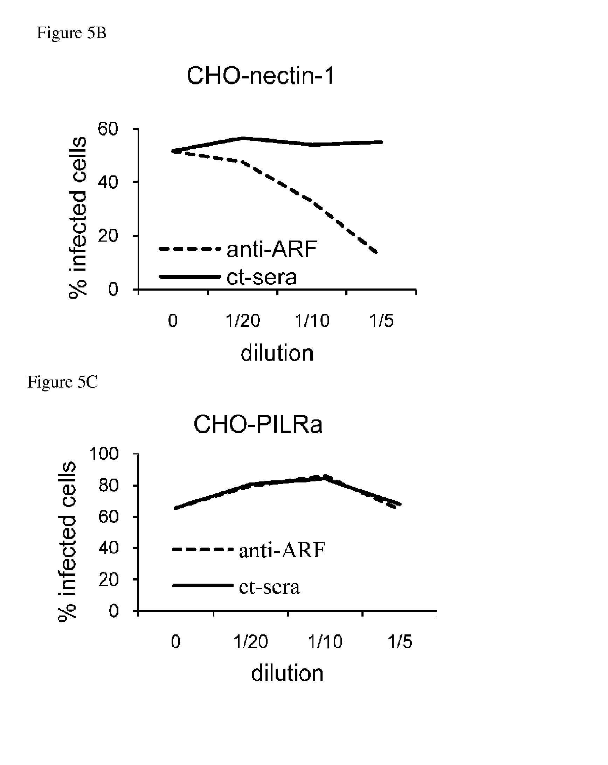

Vero cells, CHO-hNectin-1 cells, CHO-hPILRα cells, and HL60/NMHC-IIA cells were pretreated for 30 minutes with various concentrations of anti-NMHC-IIA serums or control serums.
On a 24-well plate, Vero cells or CHO-hNectin-1 cells were inoculated with HSV-1 GFP at MOI=1. After virus adsorption for one hour, the inoculum was removed and a proper medium was added to the cells.
Separately, on a 24-well plate, CHO-hPILRα cells were inoculated with HSV-1 GFP at MOI=1, followed by centrifugation at 32°C at 1100 x g for one hour. After virus adsorption for one hour, the inoculum was removed, the cells were washed, and a proper medium was added thereto.
Five hours, six hours, or 12 hours after infection, the cells were analyzed using a fluorescence microscope (Olympus IX71) or analyzed by FACSCalibur while using Cell Quest software (Becton Dickinson).
The results are shown in FIG. 5.
The anti-NMHC-IIA serum inhibited the infection of HSV-1 to CHO-hNectin-1 cells. The CHO-hNectin-1 cells are obtained by converting CHO-K1 cells having originally resistance to HSV-1 infection into HSV-1 sensitive cells by transducing nectin-1, a gD receptor, to the cells. On the contrary, the anti-NMHC-IIA serum did not inhibit the infection of HSV-1 to CHO-hPILRα cells, which is presumed to occur because CHO-K1 cells acquire sufficient sensitivity to HSV-1 by overexpression of PILRα which is the other gB receptor.
CHO-hNectin-1 and CHO-hPILRα cells each endogenously express NMHC-IIA. A competitive result in these CHO-hPILRα cells supports the conclusion that NMHC-IIA is a functional gB receptor for HSV-1.

### Example 4-2: Inhibition of HSV-1 infection by anti-NMHC-IIA antibody and anti-MNHC-IIB antibody

A test was made according to the method of Example 4-1 by using, as an antibody, an anti-NMHC-IIArod mouse serum and an anti-NMHC-IIB mouse serum. Vero cells were used and the antibody was diluted to 1:20.
The results are shown in FIG. 23. The anti-NMHC-IIArod mouse serum and the anti-NMHC-IIBrod mouse serum each inhibited HSV-1 infection.

### Example 4-3: Inhibition of HSV-2 infection by anti-NMHC-IIA antibody and anti-MNHC-IIB antibody

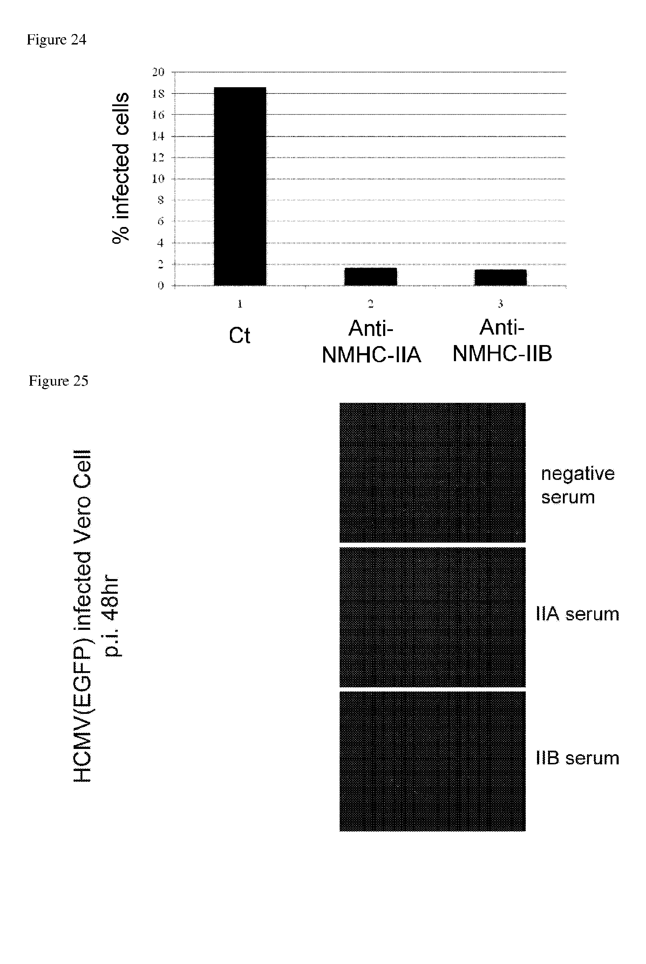

A test was made according to the method of Example 4-1 by using HSV-2 GFP as a virus and an anti-NMHC-IIArod mouse serum and an anti-NMHC-IIB mouse serum as an antibody. Vero cells were used and the antibody was diluted to 1:20.
The results are shown in FIG. 24. The anti-NMHC-IIArod mouse serum and the anti-NMHC-IIBrod mouse serum each inhibited HSV-2 infection.

### Example 5-1: Inhibition of HSV-1 infection by NMHC-IIA or NMHC-IIB knockdown

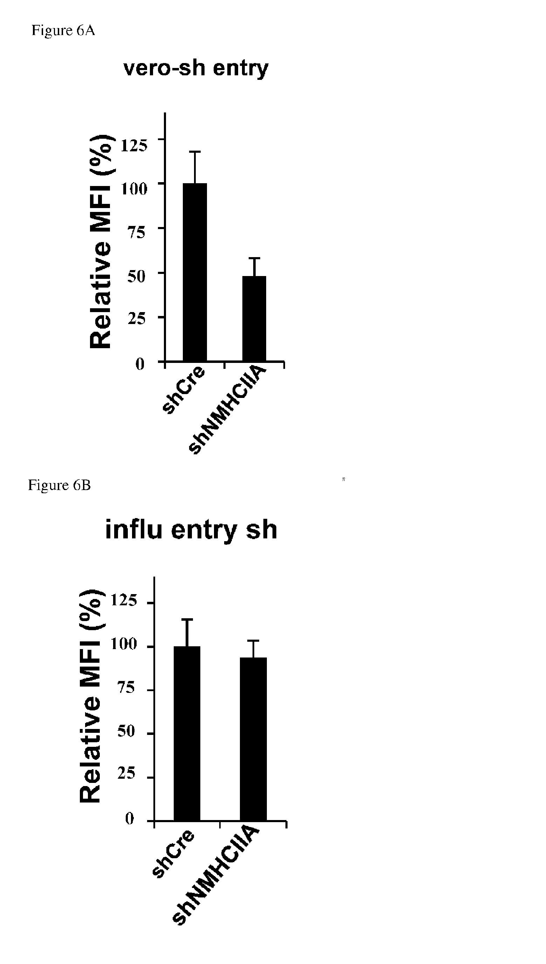

Expression of NMHC-IIA was knocked down by RNAi and an influence on virus infection was investigated.
More specifically, Vero cells were transfected with pSSSP-NMHC-IIA or pSSSP-Cre. Then, 2.5 µg/ml puromycin was added to a maintenance medium and the transfected cells were selected. The puromycin-resistant cells transduced by pSSSP-Cre were named Vero-shCre. Single colonies transduced by pSSSP-NMHC-IIA were isolated and screened by immunoblotting with an anti-NMHC-IIA antibody to select cells stably expressing shRNA against NMHC-IIA.
On a 24-well plate, Vero-shCre or Vero-NMHC-IIA cells were inoculated with HSV-1 GFP or an influenza virus at MOI=1. After viral adsorption for one hour, the inoculum was removed and a proper medium was added to the cells.
Six hours (HSV-1) or seven hours (influenza virus) after infection, the cells were analyzed by FacsCalibur while using Cell Quest software (Becton Dickinson).
The results are shown in FIGS. 6A and 6B.
The sensitivity of HSV-1 GFP to the NMHC-IIA knockdown cells (Vero-shNMHC-IIA) decreased compared with that to the cells (Vero-shCre) expressing irrelevant shRNA (FIG. 6A). On the other hand, NMHC-IIA knockdown had almost no influence on the influenza virus infection (FIG. 6B).
The results of Example 4 and Example 5 have revealed that in the cells endogenously expressing NMHC-IIA, HSV-1 makes use of NMHC-IIA as a functional receptor.
Vero cells endogenously express NMHCII-A and Nectin-1 which is a gD receptor (Milne, R. S. et al. Virology 281, 315-328 (2001).).
It has already been known that the gD receptor on Vero cells also mediate HSV-1 infection. In addition, it has been proved that binding of gB to NMHC-IIA is necessary for the infection of Vero cells with HSV-1. This means that a hypothesis that both receptors for gD and gB are necessary for the entry of HSV-1 is true.

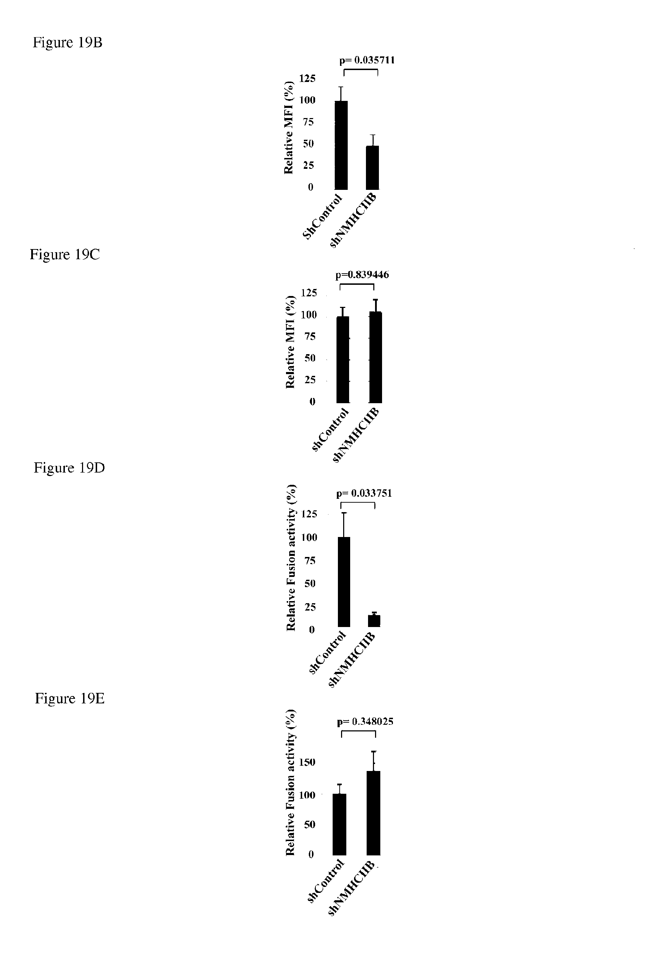

Next, the expression of NMHC-IIB in Cos-1 cells was knocked down by RNAi. More specifically, Cos-1 cells were transfected with pSSSP-NMHC-IIB or pSSSP-Cre, 2.5 µg/ml puromycin was added to a maintenance medium, and the cells thus transduced were selected. The puromycin-resistant cells transduced with pSSSP-Cre were named "Cos-1-shControl". Single colonies tranceduced with pSSSP-NMHC-IIB were isolated and were screened by immunoblotting with an anti-NMHC-IIB antibody to select cells stably expressing shRNA against NMHC-IIB.
On a 24-well plate, Cos-1-shControl (shControl in the Figure) or Cos-1-NMHC-IIB cells (shNMHC-IIB in the Figure) were inoculated with HSV-1 GFP or an influenza virus at MOI=1. After viral adsorption for one hour, the inoculum was removed and a proper medium was added to the cells.
Six hours (HSV-1) or seven hours (influenza virus) after the infection, the cells were analyzed by FACSCalibur while using Cell Quest software (Becton Dickinson).
The results are shown in FIGS. 19A to 19C. Knockdown of NMHC-IIB (FIG. 19A) decreased HSV-1 infection (FIG. 19B) but had no influence on the influenza virus infection.

### Example 5-2: Inhibition of HSV-2 infection by knockdown of NMHC-IIA or NMHC-IIB

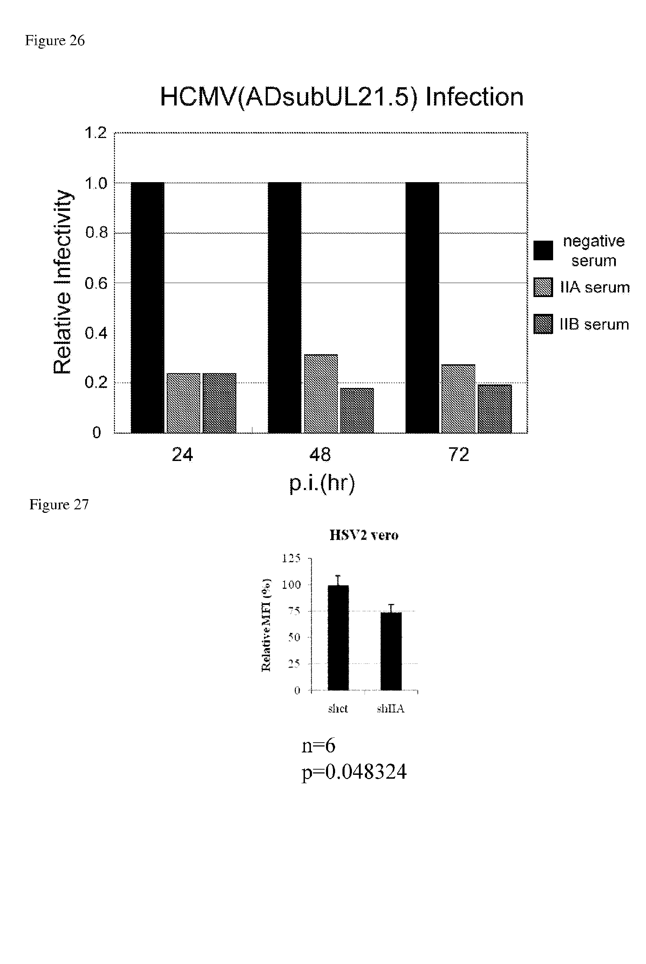

According to the method employed in Example 5-1, Vero cells in which expression of NMHC-IIA had been knocked down were inoculated with HSV-2 GFP at MOI=1. As a control, Vero-shCre was used.
The results are shown in FIG. 27. It has been confirmed that in the NMHC-IIA knockdown cells, HSV-2 infection is suppressed.

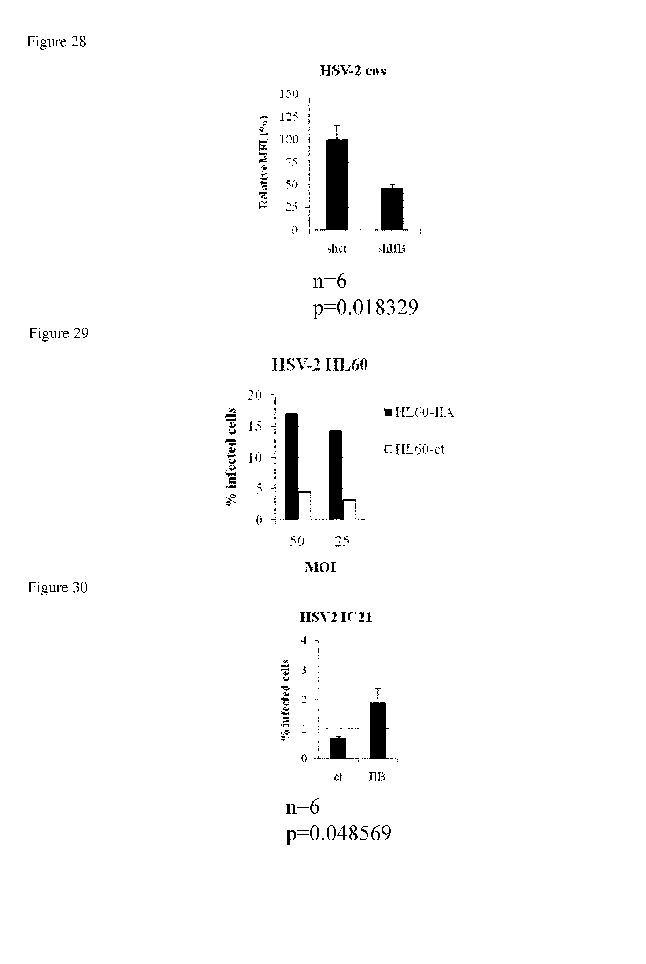

Similarly, according to the method employed in Example 5-1, Cos-1 cells in which expression of NMHC-IIB had been knocked down were inoculated with HSV-2 GFP at MOI=1.
The results are shown in FIG. 28. It has been confirmed that also in the NMHC-IIB knockdown cells, HSV-2 infection is suppressed.

### Example 6: Membrane fusion assay

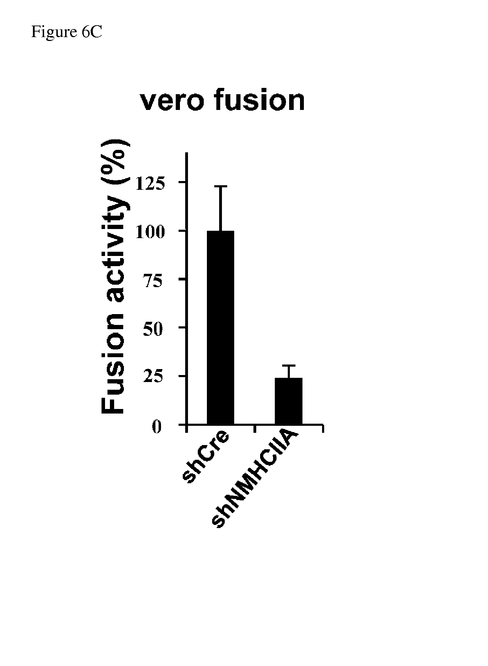

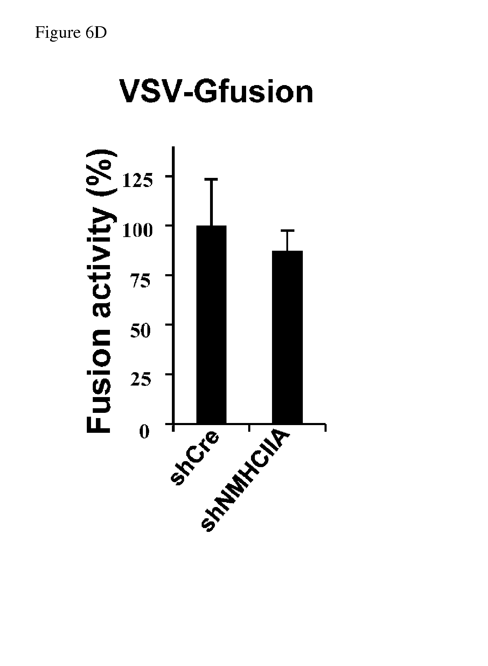

Envelope viruses such as HSV-1 require fusion between the envelope and a cell membrane of a host cell to accomplish virus infection. In order to find the role of NMHC-IIA in the membrane fusion with HSV-1, membrane fusion assay was performed.
Described specifically, on a 24-well plate, Vero cells were transfected with pEP98-gB, pPEP99-gD, pPEP101-gL, pPEP100-gH(6C) or pMD (VSV-G expression vector), and pCAGT7 and the transfectants thus obtained were used as effector cells.
Vero-shCre or Vero-shNMHC-IIA 1-3 cells in a 24-well plate were transfected with pT7EMCLuc and the transfectants thus obtained were used as target cells.
As an internal control, pRL-CMV (Promega) encoding a Renilla luciferase gene driven by a CMV promoter was cotransfected into target cells.
Six hours after transfection, the effector cells were detached by 0.04% EDTA (in PBS), washed once with a maintenance medium, and co-cultured for 18 hours with the target cells. Then, firefly luciferase and Renilla luciferase activities were independently quantified by using Dual-Luciferase Reporter Assay System (Promega) and luminometer (Promega). The firefly luciferase activity was normalized with Renilla luciferase.
The results are shown in FIGS. 6C and 6D.
When NMHC-IIB knockdown Vero cells were co-cultured with Vero cells transiently expressing HSV-1 gB, gD, gH, and gL, membrane fusion obviously decreased compared with the case where Vero cells expressing control shRNA were co-cultured with Vero cells expressing HSV-1 glycoproteins (FIG. 6C).
In contrast, knockdown of NMHC-IIA had almost no influence on the membrane fusion mediated by the VSV envelope G protein (FIG. 6D). These results show that NMHC-IIA is necessary for efficient membrane fusion mediated by the binding with glycoprotein of HSV-1 envelope. It has also been suggested that interaction between gB and NMHC-IIA is involved in the membrane fusion in HSV-1 infection.

Similarly, membrane fusion assay was performed in order to study the role of NMHC-IIB in the membrane fusion with HSV-1.
Described specifically, on a 24-well plate, Cos-1 cells were transfected with pEP98-gB, pPEP99-gD, pPEP101-gL, pPEP100-gH(6C) or pMD (VSV-G expression vector), and pCAGT7 and the transfectants were used as effector cells.
Cos-1-shControl or Cos-1-shNMHC-IIB 1-3 cells in a 24-well plate were transfected with pT7EMCLuc and the transfectants thus obtained were used as target cells.
As an internal control, pRL-CMV (Promega) encoding Renilla luciferase gene driven by a CMV controller was cotransfected into target cells.
Six hours after transfection, the effector cells were detached in 0.04% EDTA (in PBS), washed once with a maintenance medium, and co-cultured for 18 hours with target cells. Then, firefly luciferase and Renilla luciferase activities were independently determined by using Dual-Luciferase Reporter Assay System (Promega) and luminometer (Promega). The firefly luciferase activity was normalized with Renilla luciferase.
The results are shown in FIGS. 19D and 19E. When NMHC-IIB knockdown Cos-1 cells were co-cultured with Cos-1 cells co-expressing HSV-1 gB, gD, gH, and gL, membrane fusion markedly decreased compared with control (FIG. 19D). On the other hand, knockdown of NMHC-IIB had no influence on the VSV-G dependent membrane fusion. These results show that NMHC-IIB is also necessary for efficient membrane fusion mediated by the binding with the HSV-1 envelope glycoproteins. It has also been suggested that interaction between gB and NMHC-IIB is involved in the membrane fusion in HSV-1 infection.

### Example 7-1: Confirmation of involvement of NMHC-IIA in entry of HSV-1 into cells

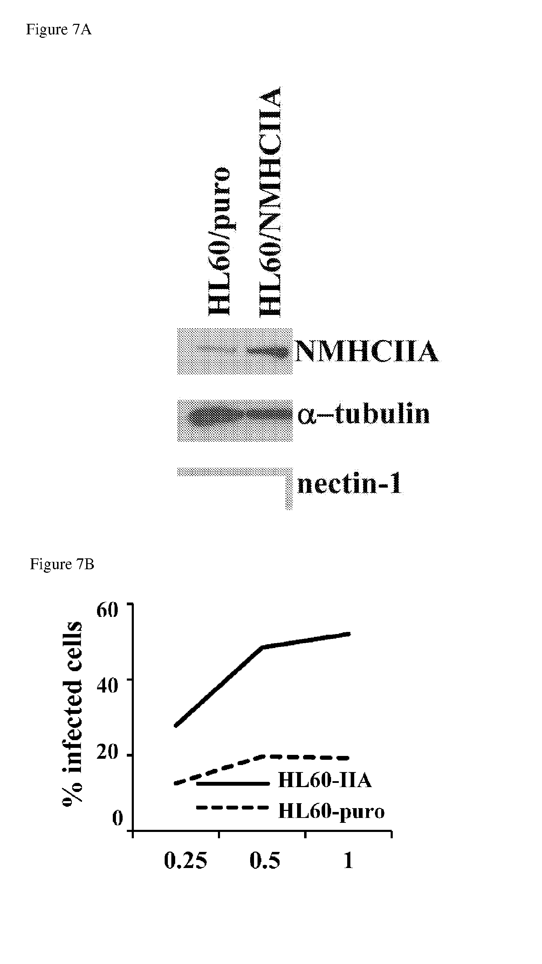

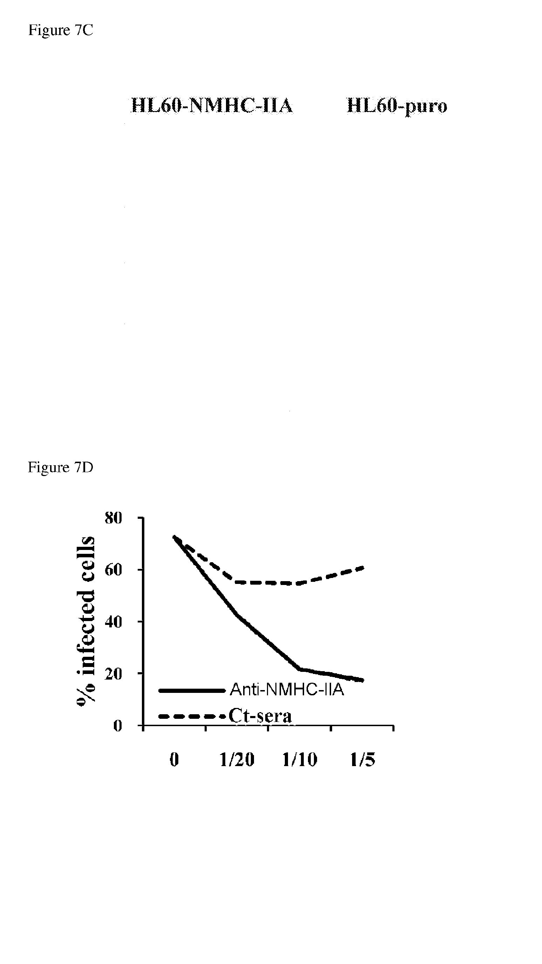

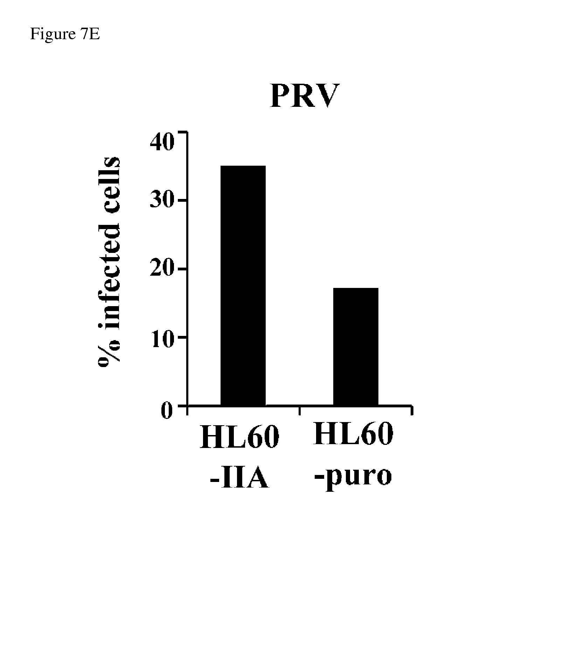

HL60 cells stably expressing a high level of NMHC-IIA (HL60/NMHC-IIA) were established. It has been reported that in human promyelocytic HL60 cells, an expression level of NMHC-IIA is low (Toothaker, L. E. et al. Blood 78, 1826-1833 (1991).) and they are relatively resistant to HSV-1 infection (Pientong, C. et al. Virology 170, 468-476 (1989)).
On a 24-well plate, HL60/NMHC-IIA cells were inoculated with HSV-1 GFP at MOI=1, followed by centrifugation at 32°C at 1100 x g for one hour. After virus adsorption for one hour, the inoculum was removed, the cells were washed, and a proper medium was given thereto.
Five hours, six hours, or twelve hours after infection, the cells were analyzed by using a fluorescence microscope (Olympus IX71) or analyzed by FACSCalibur while using Cell Quest software (Becton Dickinson).
The results are shown in FIG. 7.
FIG. 7A shows the confirmation results of overexpression of NMHC-IIA in HL60/NMHC-IIA cells by using western blotting.
The overexpression of NMHC-IIA in H60 cells markedly increased a percentage of HL60/NMHC-IIA cells infected with HSV-1 GFP compared with the HL60/puro cells used as a control (FIGS. 7B and 7C).
Infection of HL60/NMHC-IIA cells with HSV-1 GFP was dose-independently inhibited by an anti-NMHC-IIA serum, while the control serum had only a slight influence on the infection of the cells with HSV-1 GFP (FIG. 7D).
Moreover, overexpression of NMHC-IIA also enhances the sensitivity of HL60 cells to porcine alphaherpesvirus and infection with pseudorabies virus expressing GFP (PRV GFP) and infection of HL60/NMHC-IIA cells with PRV GFP were inhibited specifically by an anti-NMHC-IIA serum (FIG. 7E).
These results have suggested that NMHC-IIA mediates HSV-1 infection and NMHC-IIA-mediated virus entry into cells is conserved in other alphaherpesviruses.

### Example 7-2: Confirmation of involvement of NMHC-IIB in entry of HSV-1 into cells

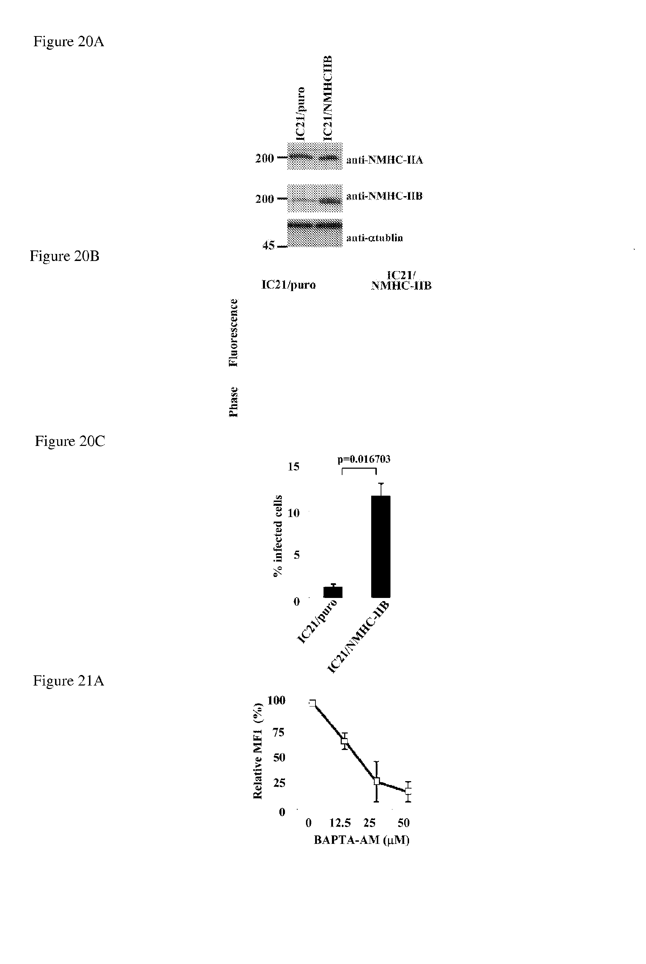

In IC21 cells having an originally low NMHC-IIB expression level, NMHC-IIB were overexpressed (FIG. 20A). These IC21/NMHC-IIB cells were inoculated with HSV-1 GFP and the resulting cells were analyzed in a similar manner to that employed for HL60/NMHC-IIA.
The results are shown in FIG. 20B. The overexpression of NMHC-IIB in IC21 cells markedly increased a percentage of cells infected with HSV-1 GFP compared with control (IC21/puro cells) (FIGS. 20B and 20C). These results show that also NMHC-IIB mediates HSV-1 infection.

### Example 7-3: Confirmation of involvement of NMHC-IIA in entry of HSV-2 in cells

With HSV-2 GFP as a virus, a similar test to Example 7-1 was conducted. The results are shown in FIG. 29. The overexpression of NMHC-IIA increased a percentage of cells infected with HSV-2 GFP. These results show that NMHC-IIA also mediates HSV-2 infection.

### Example 7-4: Confirmation of involvement of NMHC-IIB in entry of HSV-2 into cells

With HSV-2 GFP as a virus, a similar test to Example 7-3 was conducted. The results are shown in FIG. 30. The overexpression of NMHC-IIB increased a percentage of cells infected with HSV-2 GFP. These results show that NMHC-IIB also mediates HSV-2 infection.

### Example 8: Comparison with aciclovir in terms of the site of action

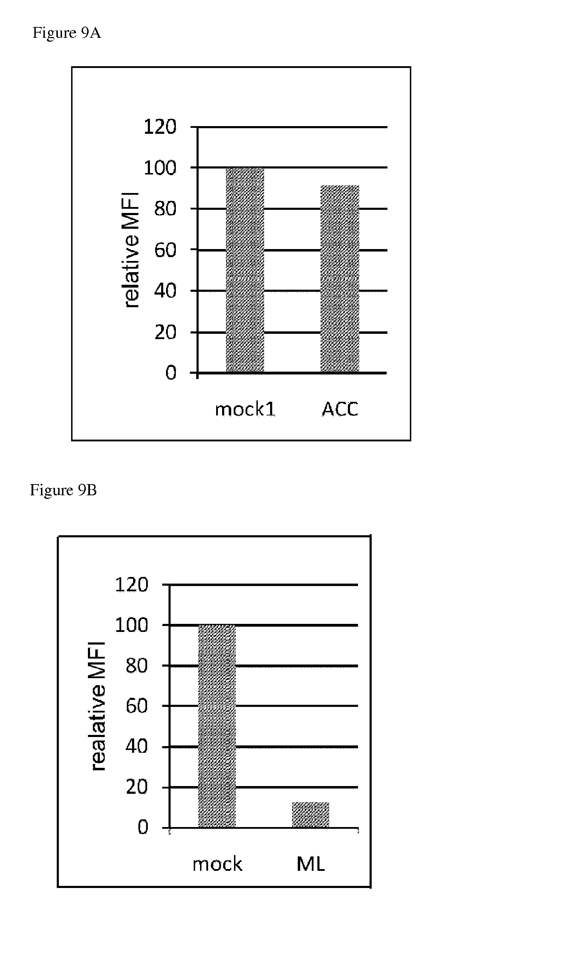

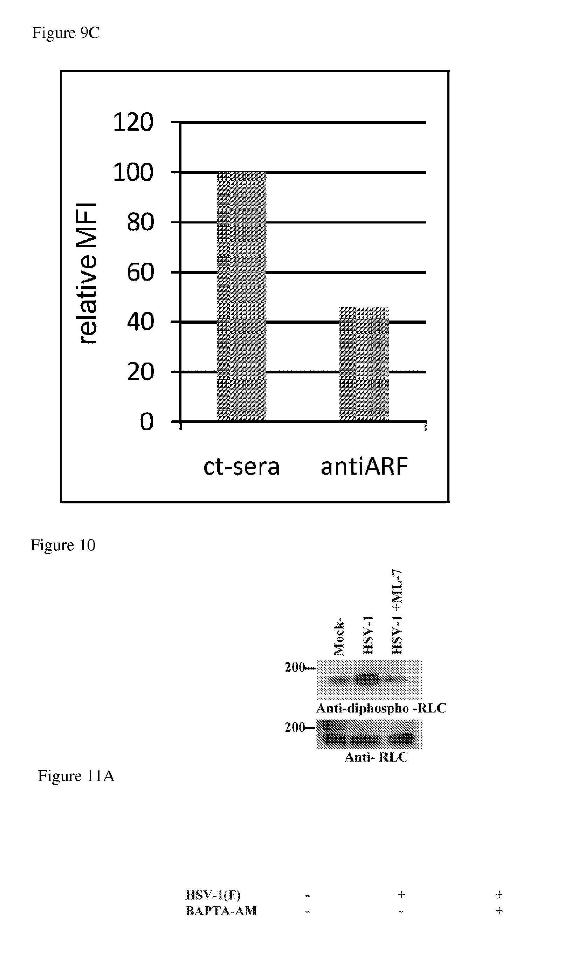

Vero cells in a 24 well plate were treated respectively with an anti-NMHC-IIA serum or a control serum, ML-7 (20 µM), and ACC (40 µM) for 30 minutes and inoculated with HSV-1 GFP at MOI=1. One hour later, the virus solution was removed and a maintenance medium was added. The cells treated with ML-7 and ACC were cultured on maintenance media containing the drugs, respectively. Six hours after infection, the cells were harvested and their fluorescence intensity was analyzed using a flow cytometer. Relative fluorescence intensity is shown in Figures.
Although HSV entry into Vero cells was not inhibited by ACC, a HSV replication inhibitor (FIG. 9A), it was inhibited by the MLCK inhibitor ML-7 (FIG. 9B) or the NMHC-IIA antiserum (FIG. 9C). ACC is an anti-herpesvirus drug which has already been used widely but the above results suggest that the drug or antibody targeting NMHC-IIA has the site of action utterly different from ACC.

### Example 9: Phosphorylation of RLC caused by HSV-1 infection

Vero cells were mock treated or treated for 30 minutes with 20 µM ML-7 and then mock-exposed or exposed to wild-type HSV-1 at MOI=50 in the presence or absence of ML-7 at 4°C for 2 hours. Fifteen minutes after transfer of the resulting cells to 37°C, phosphorylation of RLC was measured by immunoblotting using an anti-phosphorylated RLC antibody or an anti-RLC antibody.
The results are shown in FIG. 10. It has been confirmed that HSV-1 infection enhances phosphorylation of RLC and ML-7, a selective inhibitor of MLCK, inhibits this enhancement.

### Example 10: Suppression of HSV-1 infection by calcium chelator

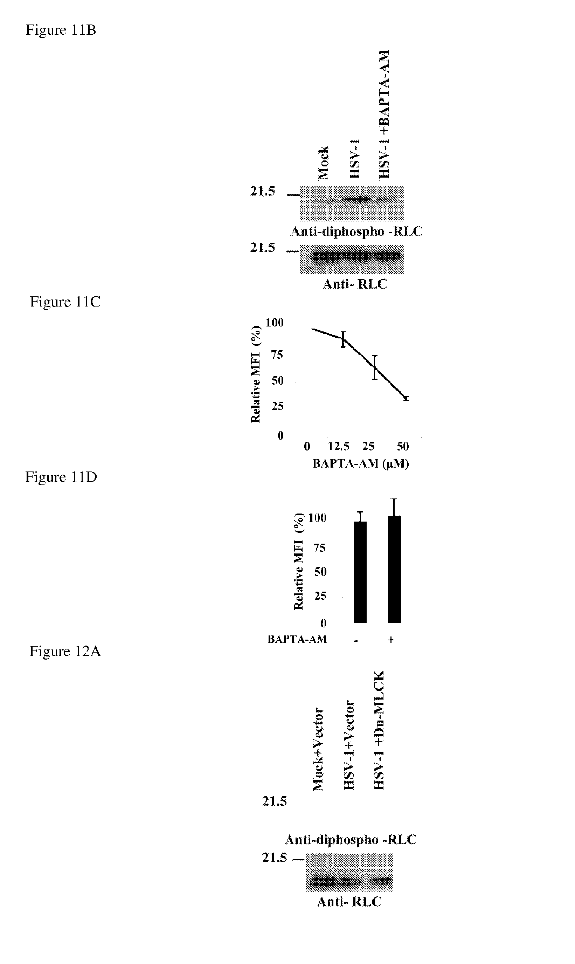

Vero cells were mock treated or treated for 30 minutes with 50 µM BAPTA-AM and then mock-exposed or exposed to wild-type HSV-1 at MOI=50 in the presence or absence of ML-7 at 4°C for 2 hours. Fifteen minutes after transfer of the resulting cells to 37°C, they were analyzed using the immunofluorescent method using an anti-NMHC-IIA antibody.
The results are shown in FIG. 11A. BAPTA-AM is a Ca²⁺ chelator indispensable for the activation of MLCK. It has been confirmed that in the presence of BAPTA-AM, even if the cells are infected with HSV-1, translocation of NMHC-IIA to the vicinity of the cell membrane does not occur.
Vero cells were mock treated or treated with 50 µM BAPTA-AM for 30 minutes and then mock exposed or exposed to wild type HSV-1 at MOI=5 in the presence or absence of ML-7 at 4°C for 2 hours. Fifteen minutes after transfer to 37°C, expression and phosphorylation of RLS were measured using immunoblotting.
The results are shown in FIG. 11B. It has been confirmed that in the presence of BAPTA-AM, there was no change in the expression amount of RLC, but enhancement of phosphorylation of RLC due to HSV-1 infection was suppressed.
Next, Vero cells were infected with HSV-1 GFP at MOI=1 in the presence or absence of BAPTA-AM at the indicated concentrations. Five hours after infection, mean fluorescence intensity (MFI) was measured using flow cytometry. The data are presented as average with standard deviation (n=3). The data were normalized with the value measured in the absence of BAPTA-AM.
The results are shown in FIG. 11C. The MFI decreased in a BAPTA-AM concentration dependent manner. This suggests that inhibition of the activity of MLCK by a calcium chelator leads to a decrease in HSV-1 entrering into cells.
On the other hand, Vero cells were infected with an influenza virus at MOI=1 in the presence or absence of 50 µM BAPTA-AM. Seven hours after infection, MFI was measured using flow cytometry. Data are presented as average and standard deviation (n=3). The average in the absence of BAPTA-AM was normalized with 100% relative MFI.
The results are shown in FIG. 11D. It has been confirmed that relative MFI was not influenced by the presence/absence of BAPTA-AM and influenza virus infection was not suppressed by a calcium chelator.

Next, Cos-1 cells instead of Vero cells were infected with HSV-1 GFP at MOI=1 in the presence or absence of BAPTA-AM at various concentrations. Similar to the case of Vero cells, fluorescence intensity was measured. The results are shown in FIG. 21A. MFI decreased in a BAPTA-AM concentration dependent manner. This shows that also in the cells expressing only NMHC-IIB, inhibition of MLCK activity by a calcium chelator leads to a decrease in HSV-1 entering into the cells. When the cells were infected with an influenza virus instead of HSV-1, influence of the calcium chelator on the infection was not observed.

### Example 11: Suppression of HSV-1 infection by dominant negative mutant of MLCK

Vero cells transformed with a mock expression plasmid (Vector) or an expression plasmid of dominant negative mutant of MLCK (Dn-MLCK) were mock incubated at 4°C for 2 hours or exposed to wild-type HSV-1 at MOI=50.
Fifteen minutes after the cells were transferred to 37°C, expression and phosphorylation of RLC were measured by immunoblotting using an anti-phosphorylated RLC antibody or an anti-RLC antibody. The results are shown in FIG. 12A. Data show typical examples of three independent experiments.
Vector was pCI-neo purchased from Promega. As the dominant negative mutant expression plasmid, that described in J. Physiol 570: 219-235, 2006 was used.
Phosphorylation of RLC increased due to HSV-1 infection in the cells transformed with Vector, while phosphorylation increase was suppressed in the cells transformed with the dominant negative mutant.

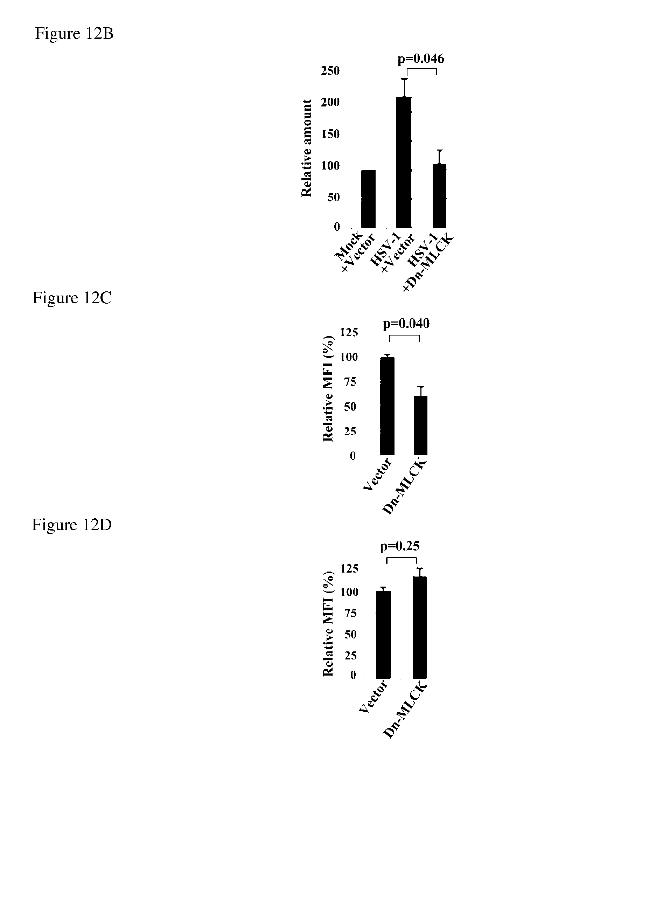

FIG. 12B shows the results, determined from the above-mentioned results, of a relative amount of phosphorylated RLC protein to the total RLC protein mass. The relative amount is a phosphorylation amount of RLC in cells transformed with Vector or Dn-MLCK, followed by HSV-1 infection (HSV-1 + Vector or HSV-1+Dn-MLCK, respectively), which is determined assuming that the phosphorylation amount of RLC in cells transformed with Vector, followed by mock infection (Mock + Vector) is 100. The data are presented as average and standard deviation (n=3, two-tailed Student's t-test). It has been confirmed that in HSV-1+Vector, phosphorylation of RLC has been markedly enhanced, while enhancement of the phosphorylation is almost suppressed by the dominant negative mutant of MLCK.
Next, Vero cells transformed with Vector or Dn-MLCK were infected with wild-type HSV-1 GFP (FIG. 12C) or with an influenza virus (FIG. 12D) at MOI=1. Five hours or seven hours after infection, the cells were analyzed by using flow cytometry to determine mean fluorescence intensity (MFI). Data are presented as average and standard deviation (n=3, two-tailed Student's t-test). The average in the cells infected with Vector was normalized with 100% relative MFI.
Compared with the case where the cells transformed with Vector were infected with HSV-1, the entry of HSV-1 in the cells transformed with Dn-MLCK was about 60%, from which it has been confirmed that entry of HSV-1 was markedly suppressed. When the cells transformed with Dn-MLCK were infected with an influenza virus, on the other hand, entry of an influenza virus rather increased.
The results of Examples 9 to 10 show further that a change in intracellular localization of NMHC-IIA or HSV infection is controlled by MLCK signal and therefore, HSV-1 infection can be suppressed by inhibiting this MLCK signal.

### Example 12: Effect of MLCK inhibitor on murine corneal infection model (administration before infection)

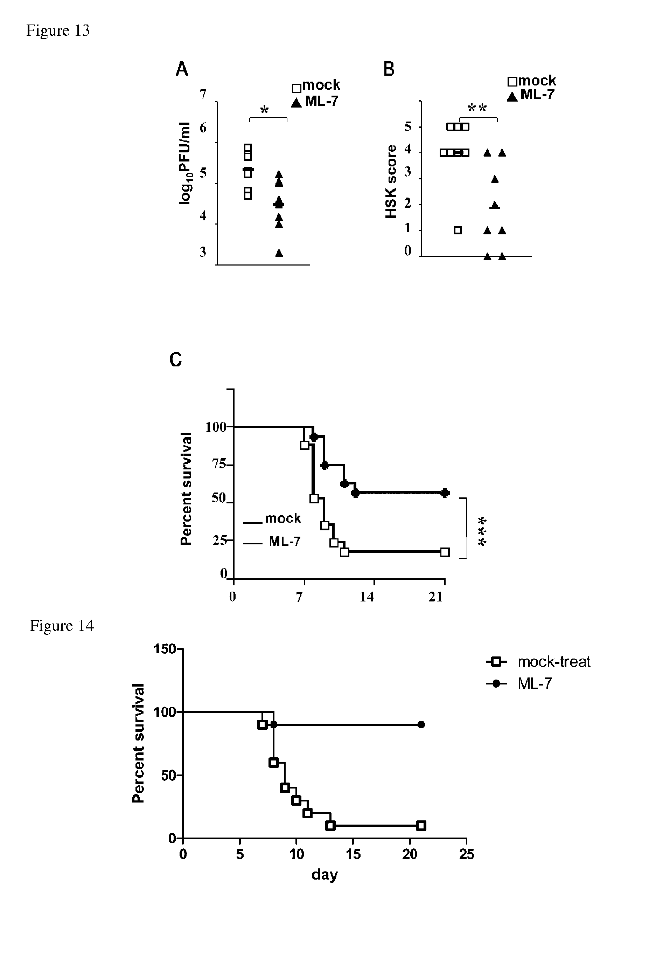

After the cornea of ICR mice (female/5-week-old) was slightly injured with an injection needle, a medium containing or not containing 20 µM ML-7 was instilled in the eye twice with an interval of 10 minutes. After incubation for 10 minutes, an HSV-1(F) strain was inoculated at 5x10⁵ PFU/eye. The virus titer in the tear, symptoms of keratitis, and survival of mice were studied.
The results are shown in FIG. 13. In the mice treated with ML-7, either of the virus titer (FIG. 13A) two days after virus inoculation and symptoms of keratitis after five days (FIG. 13B) showed a significant decrease compared with the untreated mice (A; p<0.001, B; p<0.05). Moreover, in the ML-7 treated group, the survival rate of mice showed a significant increase (FIG. 13C).
These results have suggested that NMHC-IIA has been utilized by HSV-1 also in the living body and at the same time, there is a possibility that a drug, such as ML-7, involved in the control of NMHC-IIA can be used as a remedy/preventive, particularly preventive for herpesvirus.

### Example 13: Effect of MLCK inhibitor (ML-7) on murine corneal infection model (administration simultaneous with infection)

After the cornea of 28 ICR mice(female/5-week-old) per group was injured with an injection needle, a 20 µM medium containing or not containing ML-7 and 5×10⁵ HSV-1(F) diluted in Medium199 were simultaneously instilled in their eye, followed by observation for 21 days.
The results are shown in FIG. 14. Compared with FIG. 13, the survival rate of mice in the ML-7 treatment group shows a further significant increase.

### Example 14: Effect of MLCK inhibitor (BAPTA-AM) on murine corneal infection model

After the cornea of 28 ICR mice (female/5-week-old) per group was injured with an injection needle, a 50 µM BAPTA-AM was instilled in their eye twice with an interval of 10 minutes. After incubation for 10 minutes, 5×10⁵ HSV-1(F) diluted in Medium 199 was instilled in their eye, followed by observation for 21 days. In Mock-treated group, 0.2% DMSO in Medium 199 was used instead of 50 µM BAPTA-AM.
The results are shown in FIG. 15. Compared with the Mock-treated group, the survival rate of the BAPTA-AM-treated group was significantly high. This result shows that the MLCK inhibitor is useful as a remedy/preventive of herpesvirus infections.

### Example 15: Inhibition of cytomegalovirus (HCMV) infection by anti-NMHC-IIA antibody and anti-MNHC-IIB antibody

HEL cells were pretreated with an anti-NMHC-IIArod mouse serum, an anti-NMHC-IIBrod mouse serum, and a control serum for 30 minutes and infected with HCMV ADsubU21.5 at MOI=0.1 in the presence of an antibody. GFP expression 24 hours, 46 hours, and 72 hours after the infection was analyzed by FACS through a fluorescence microscope.
A fluorescence microscope image of GFP expression cells 48 hours after virus infection is shown in FIG. 25. In addition, FACS analysis results of GFP expression cells 24 hours, 48 hours, and 72 hours after virus infection are shown in FIG. 26.
It has been revealed that an anti-NMHC-IIA antibody and an anti-NMHC-IIB antibody inhibit HCMV infection. This suggests that an infection method via NMHC-IIA and NMHC-IIB is widely common not only to viruses belonging to subfamily *Alphaherpesvirinae* such as HSV-1, HSV-2 and PRV but also to herpesviruses belonging to another subfamily.

### Free text of sequence listing

SEQ ID NO:1 is an amino acid sequence of the C-terminal region (from position 1665 to position 1960) of NMHC-IIA.
SEQ ID NO:2 is one example of a target sequence of siRNA against NMHC-IIA.
SEQ ID NO:3 is a single strand of one example of siRNA against NMHC-IIA.
SEQ ID NO:4 is a single strand of one example of siRNA against NMHC-IIA.
SEQ ID NO:5 is a DNA encoding one example of siRNA against NMHC-IIA.
SEQ ID NO:6 is an epitope recognized by a rabbit polyclonal antibody against the C terminal of NMHC-IIA used in Example.
SEQ ID NO:7 is an amino acid sequence of the C terminal region (from position 1672 to position 1976) of NMHC-IIB.
SEQ ID NO:8 is one example of a target sequence of siRNA against NMHC-IIB.
SEQ ID NO:9 is a single strand of one example of siRNA against NMHC-IIB.
SEQ ID NO:10 is a single chain of one example of siRNA against NMHC-IIB.
SEQ ID NO:11 is a DNA encoding one example of shRNA against NMHD-IIB.
SEQ ID NO:12 is an epitope recognized by a rabbit polyclonal antibody against the C terminal of NMHC-IIB used in Example.

## Claims

1. A pharmaceutical composition for the prevention or treatment of herpesvirus infections comprising, as an active ingredient, a substance which inhibits the binding of glycoprotein B to a non-muscle myosin heavy chain IIA or IIB.

2. The pharmaceutical composition according to Claim 1, wherein the substance inhibiting the binding of glycoprotein B to a non-muscle myosin heavy chain IIA or IIB is a myosin ATPase activity inhibitor or a myosin light chain kinase inhibitor.

3. The pharmaceutical composition according to Claim 2, wherein the myosin light chain kinase inhibitor is ML-7.

4. The pharmaceutical composition according to Claim 2, wherein the myosin light chain kinase inhibitor is an MLCK pathway inhibitor.

5. The pharmaceutical composition according to Claim 4, wherein the MLCK pathway inhibitor is selected from the group consisting of calmodulin antagonists, calcium chelators, and calcium antagonists.

6. The pharmaceutical composition according to Claim 2, wherein the myosin light chain kinase inhibitor is a dominant negative mutant of myosin light chain kinase.

7. The pharmaceutical composition according to Claim 1, wherein the substance which inhibits the binding of glycoprotein B to a non-muscle myosin heavy chain IIA or a non-muscle myosin heavy chain IIB is an antibody against the non-muscle myosin heavy chain IIA or the non-muscle myosin heavy chain IIB.

8. The pharmaceutical composition according to Claim 7, wherein the antibody binds to a peptide having an amino acid sequence as set forth in SEQ ID NO: 1 or 7.

9. The pharmaceutical composition according to Claim 7, wherein the antibody binds to a region of the non-muscle myosin heavy chain IIA or the non-muscle myosin heavy chain IIB which is exposed extracellularly upon herpesvirus infection.

10. The pharmaceutical composition according to Claim 1, wherein the substance which inhibits the binding of glycoprotein B to a non-muscle myosin heavy chain IIA or a non-muscle myosin heavy chain IIB is a substance which suppresses expression of the non-muscle myosin heavy chain IIA or the non-muscle myosin heavy chain IIB.

11. The pharmaceutical composition according to Claim 10, wherein the substance which suppresses expression of the non-muscle myosin heavy chain IIA or the non-muscle myosin heavy chain IIB is selected from the group consisting of double-stranded nucleic acids having an RNAi effect, antisense nucleic acids, and ribozymes, and nucleic acids encoding them.

12. The pharmaceutical composition according to Claim 1, wherein the substance which inhibits the binding of glycoprotein B to a non-muscle myosin heavy chain IIA or a non-muscle myosin heavy chain IIB is a soluble form of the non-muscle myosin heavy chain IIA or a soluble form of the non-muscle myosin heavy chain IIB.

13. The pharmaceutical composition according to any one of Claims 1 to 12, wherein the herpesvirus is simplex herpesvirus, porcine herpesvirus 1, or cytomegalovirus;

14. A double-stranded RNA consisting of base sequences as set forth in SEQ ID NO:3 and SEQ ID NO:4 and having an RNAi effect against a non-muscle myosin heavy chain IIA;

15. A nucleic acid comprising DNA having a base sequence as set forth in SEQ ID NO:5 and encoding a double-stranded RNA having an RNAi effect against a non-muscle myosin heavy chain IIA.

16. A double-stranded RNA consisting of base sequences as set forth in SEQ ID NO:9 and SEQ ID NO:10 and having an RNAi effect against a non-muscle myosin heavy chain IIB.

17. A nucleic acid comprising DNA having a base sequence as set forth in SEQ ID NO:11 and encoding a double-stranded RNA having an RNAi effect against a non-muscle myosin heavy chain IIB.

18. A method of screening for a pharmaceutical for the prevention or treatment of herpesvirus infections, comprising:
treating cells expressing a non-muscle myosin heavy chain IIA or a non-muscle myosin heavy chain IIB with candidate compounds;
infecting the cells with herpesvirus; and
measuring at least one of translocation, in the cells, of the non-muscle myosin heavy chain IIA or the non-muscle myosin heavy chain IIB to the vicinity of a cell membrane or entry of herpesvirus into the cells.

19. A method of screening for a pharmaceutical for the prevention or treatment of herpesvirus infections, comprising:
bringing a non-muscle myosin heavy chain IIA or a non-muscle myosin heavy chain IIB, gB, and candidate compounds into contact with each other under conditions permitting binding of the non-muscle myosin heavy chain IIA or the non-muscle myosin heavy chaing IIB to gB, and
measuring the binding of the non-muscle myosin heavy chain IIA or the non-muscle myosin heavy chain IIB to gB.


SEQUENCE LISTING

<110>  The University of Tokyo

<120>  Pharmaceutical Composition for Treatment or Prevention of Herpes
Viral Infectious Disease

<130>  T0529ACP11

<150>  JP 2010-072717
<151>  2010-03-26

<150>  JP 2010-193101
<151>  2010-08-31

<150>  JP 2010-229537
<151>  2010-10-12

<160>  12

<170>  PatentIn version 3.1

<210>  1
<211>  296
<212>  PRT
<213>  Mus musculus

<220>
<221>  MISC_FEATURE
<223>  C terminus region of NMHC-IIA protein


<400>  1

Leu Ala Gln Ala Lys Glu Asn Glu Lys Lys Leu Lys Ser Met Glu Ala
1               5                   10                  15


Glu Met Ile Gln Leu Gln Glu Glu Leu Ala Ala Ala Glu Arg Ala Lys
            20                  25                  30


Arg Gln Ala Gln Gln Glu Arg Asp Glu Leu Ala Asp Glu Ile Ala Asn
        35                  40                  45


Ser Ser Gly Lys Gly Ala Leu Ala Leu Glu Glu Lys Arg Arg Leu Glu
    50                  55                  60


Ala Arg Ile Ala Gln Leu Glu Glu Glu Leu Glu Glu Glu Gln Gly Asn
65                  70                  75                  80


Thr Glu Leu Ile Asn Asp Arg Leu Lys Lys Ala Asn Leu Gln Ile Asp
                85                  90                  95


Gln Ile Asn Thr Asp Leu Asn Leu Glu Arg Ser His Ala Gln Lys Asn
            100                 105                 110


Glu Asn Ala Arg Gln Gln Leu Glu Arg Gln Asn Lys Glu Leu Lys Val
        115                 120                 125

Lys Leu Gln Glu Met Glu Gly Thr Val Lys Ser Lys Tyr Lys Ala Ser
    130                 135                 140

Ile Thr Ala Leu Glu Ala Lys Ile Ala Gln Leu Glu Glu Gln Leu Asp
145                 150                 155                 160

Asn Glu Thr Lys Glu Arg Gln Ala Ala Cys Lys Gln Val Arg Arg Thr
                165                 170                 175

Glu Lys Lys Leu Lys Asp Val Leu Leu Gln Val Asp Asp Glu Arg Arg
            180                 185                 190

Asn Ala Glu Gln Tyr Lys Asp Gln Ala Asp Lys Ala Ser Thr Arg Leu
        195                 200                 205

Lys Gln Leu Lys Arg Gln Leu Glu Glu Ala Glu Glu Glu Ala Gln Arg
    210                 215                 220

Ala Asn Ala Ser Arg Arg Lys Leu Gln Arg Glu Leu Glu Asp Ala Thr
225                 230                 235                 240

Glu Thr Ala Asp Ala Met Asn Arg Glu Val Ser Ser Leu Lys Asn Lys
                245                 250                 255

Leu Arg Arg Gly Asp Leu Pro Phe Val Val Pro Arg Arg Met Ala Arg
            260                 265                 270

Lys Gly Ala Gly Asp Gly Ser Asp Glu Glu Val Asp Gly Lys Ala Asp
        275                 280                 285

Gly Ala Glu Ala Lys Pro Ala Glu
    290                 295

<210>  2
<211>  21
<212>  DNA
<213>  Mus musculus

<220>
<221>  misc_feature
<223>  siRNA target region of NMHC-IIA gene

<400>  2
gaccagaact gcaagctggc c                                                21

<210>  3
<211>  21
<212>  RNA
<213>  Artificial

<220>

<223>  one strand of siRNA against NMHC-IIA gene

<400>  3
gaccagaacu gcaagcuggc c                                                21


<210>  4
<211>  21
<212>  RNA
<213>  Artificial

<220>
<223>  one strand of siRNA against NMHC-IIA gene

<400>  4
ggccagcuug caguucuggu c                                                21


<210>  5
<211>  60
<212>  DNA
<213>  Artificial

<220>
<223>  DNA coding shRNA against NMHC-IIA gene

<400>  5
gaccagaact gcaagctggc cgcttcctgt cacggccagc ttgcagttct ggtctttttt      60


<210>  6
<211>  11
<212>  PRT
<213>  Mus musculus

<220>
<221>  MISC_FEATURE
<223>  epitope recognized by rat polyclonal antibody agaisnt C-terminal
       of NMHC-IIA


<400>  6

Lys Ala Asp Gly Ala Glu Ala Lys Pro Ala Glu
1               5                   10


<210>  7
<211>  305
<212>  PRT
<213>  Mus musculus

<220>
<221>  MISC_FEATURE
<223>  C terminus region of NMHC-IIB protein


<400>  7

Phe Ala Gln Ser Lys Glu Ser Glu Lys Lys Leu Lys Ser Leu Glu Ala
1               5                   10                  15


Glu Ile Leu Gln Leu Gln Glu Glu Leu Ala Ser Ser Glu Arg Ala Arg
            20                  25                  30

Arg His Ala Glu Gln Glu Arg Asp Glu Leu Ala Asp Glu Ile Thr Asn
        35                  40                  45

Ser Ala Ser Gly Lys Ser Ala Leu Leu Asp Glu Lys Arg Arg Leu Glu
    50                  55                  60

Ala Arg Ile Ala Gln Leu Glu Glu Glu Leu Glu Glu Glu Gln Ser Asn
65                  70                  75                  80

Met Glu Leu Leu Asn Asp Arg Phe Arg Lys Thr Thr Leu Gln Val Asp
                85                  90                  95

Thr Leu Asn Ala Glu Leu Ala Ala Glu Arg Ser Ala Ala Gln Lys Ser
            100                 105                 110

Asp Asn Ala Arg Gln Gln Leu Glu Arg Gln Asn Lys Glu Leu Lys Ala
        115                 120                 125

Lys Leu Gln Glu Leu Glu Gly Ala Val Lys Ser Lys Phe Lys Ala Thr
    130                 135                 140

Ile Ser Ala Leu Glu Ala Lys Ile Gly Gln Leu Glu Glu Gln Leu Glu
145                 150                 155                 160

Gln Glu Ala Lys Glu Arg Ala Ala Ala Asn Lys Leu Val Arg Arg Thr
                165                 170                 175

Glu Lys Lys Leu Lys Glu Ile Phe Met Gln Val Glu Asp Glu Arg Arg
            180                 185                 190

His Ala Asp Gln Tyr Lys Glu Gln Met Glu Lys Ala Asn Ala Arg Met
        195                 200                 205

Lys Gln Leu Lys Arg Gln Leu Glu Glu Ala Glu Glu Glu Ala Thr Arg
    210                 215                 220

Ala Asn Ala Ser Arg Arg Lys Leu Gln Arg Glu Leu Asp Asp Ala Thr
225                 230                 235                 240

Glu Ala Asn Glu Gly Leu Ser Arg Glu Val Ser Thr Leu Lys Asn Arg
                245                 250                 255

Leu Arg Arg Gly Gly Pro Ile Ser Phe Ser Ser Ser Arg Ser Gly Arg
            260                 265                 270

Arg Gln Leu His Leu Glu Gly Ala Ser Leu Glu Leu Ser Asp Asp Asp
        275                 280                 285

Thr Glu Ser Lys Thr Ser Asp Val Asn Glu Thr Gln Pro Pro Gln Ser
    290                 295                 300

Glu
305

<210>  8
<211>  21
<212>  DNA
<213>  Mus musculus

<220>
<221>  misc_feature
<223>  siRNA target region of NMHC-IIB gene

<400>  8
ggattccatc agaacgccat g                                                21

<210>  9
<211>  21
<212>  RNA
<213>  Artificial

<220>
<223>  one strand of siRNA against NMHC-IIB gene

<400>  9
ggauuccauc agaacgccau g                                                21

<210>  10
<211>  21
<212>  RNA
<213>  Artificial

<220>
<223>  one strand of siRNA against NMHC-IIB gene

<400>  10
cauggcguuc ugauggaauc c                                                21

<210>  11
<211>  64
<212>  DNA
<213>  Artificial

<220>
<223>  DNA coding shRNA against NMHC-IIB gene

<400>  11
tttggattcc atcagaacgc catggcttcc tgtcaccatg gcgttctgat ggaatccttt      60

tttg                                                                   64

<210>  12
<211>  12
<212>  PRT
<213>  Mus musculus

<220>
<221>  MISC_FEATURE
<223>  epitope recognized by rat polyconal antibody against C-terminal o
       f NMHC-IIB

<400>  12

Ser Asp Val Asn Glu Thr Gln Pro Pro Gln Ser Glu
1               5                   10